# EUROPEAN PATENT APPLICATION

(11) **EP 4 461 745 A1**
(43) Date of publication of application: **13.11.2024**
(21) Application number: 22919084.8
(22) Date of filing: 27.12.2022
(51) Int. Cl.: C07K 16/18, C07K 14/71, C12N 15/62, A61K 38/00, A61P 27/02, A61K 39/00

(54) **ANGIOGENESIS INHIBITOR-CONJUGATED ANTI-C3B ANTIBODY OR ANTI-C5 ANTIBODY AND USE THEREOF**

(30) Priority: 05.01.2022 KR 20220001647
(71) Applicant: Kanaph Therapeutics Inc., Seoul 04348 (KR)
(72) Inventor: CHUNG, Eu Ddeum, Seoul 04348 (KR); RYU, Soomin, Seoul 04348 (KR); KIM, Donggeon, Seoul 04348 (KR); CHANG, Jihoon, Seoul 04348 (KR); LEE, Byoung Chul, Seoul 04348 (KR)
(74) Representative: HGF
(86) International application number: PCT/KR2022/021371
(87) International publication number: WO 2023/132547

(57) **Abstract**

The present invention relates to a fusion protein comprising an anti-C3b antibody or an anti-C5 antibody and an angiogenesis inhibitor, and a composition for treating eye diseases, particularly macular degeneration, by using the same. The protein may not only inhibit complement-related pathways, but can also efficiently regulate angiogenesis. Therefore, the fusion protein dimer can be effectively used to treat and prevent complement-related diseases, particularly eye diseases such as macular degeneration, and thus has high industrial applicability.

## Description

### Technical Field

The present invention relates to a fusion protein comprising an anti-C3b antibody or an anti-C5 antibody and an angiogenesis inhibitor, and a composition for treating eye diseases, particularly macular degeneration, by using the same.

### Background Art

Macular degeneration (age-related macular degeneration, AMD) is a major cause of blindness in people over 50 years of age and is an ophthalmic disease that causes loss of central vision due to damage to the macula. Macular degeneration can be broadly divided into wet or dry macular degeneration. In the early stages, dry macular degeneration occurs, and drusen, an abnormal extracellular deposit, appears under the macula, and pigment changes occur in the retinal pigment epithelium (RPE). Wet macular degeneration causes choroidal neovascularization, pigment epithelium detachment, macular edema, retinal hemorrhage, and retinal exudation, leading to blindness through death of retinal nerve cells.

Meanwhile, the complement system is a critical component of innate immunity against microbial infection and includes a group of proteins that are normally present in an inactive state in serum. The protein is activated through the classical pathway, lectin pathway, and alternative pathway. Molecules on the surface of microorganisms can activate this pathway, resulting in the formation of a protease complex known as C3-convertase.

Activation of the complement pathway produces biologically active fragments of complement proteins that mediate inflammatory responses in leukocyte chemotaxis, activation of macrophages, neutrophils, platelets, mast cells, and endothelial cells, increased vascular permeability, cell lysis and tissue damage, for example anaphylatoxins such as C3a and C5a, and C5b to C9 membrane attack complex (MAC) are produced.

In addition, recent studies have demonstrated that complement components C3 and C5 are the major components of drusen in AMD patients (Mulling, R.F. et al., FASEB J., 14, 835-46, 2000). In addition, it has been reported in Japanese Patent No. 4897690 that some eye diseases are complement-related. However, to this day, the need for drugs to effectively treat eye diseases, particularly macular degeneration, is increasing, and research on therapeutic agents for macular degeneration is continuing.

### Detailed Description of Invention

### Technical Problem

Accordingly, the present inventors conducted research to effectively treat and prevent eye diseases, particularly macular degeneration. As a result, the present inventors have found that a fusion protein that blocks the complement-related pathway and the neovascularization pathway can be used as a therapeutic agent for macular degeneration, thereby completing the present invention.

### Solution to Problem

In one aspect of the present invention, there is provided a fusion protein comprising an antibody fragment that specifically binds to C3b (complement component 3b) or C5 (complement component 5); and a protein that specifically binds to VEGF (vascular endothelial growth factor).

In another aspect of the present invention, there is provided a fusion protein dimer in which the two fusion proteins are attached to each other.

In yet another aspect of the present invention, there is provided a polynucleotide encoding the fusion protein.

In still yet another aspect of the present invention, there is provided a vector comprising the polynucleotide.

In still yet another aspect of the present invention, there is provided a transformed host cell into which the vector has been introduced.

In still yet another aspect of the present invention, there is provided a pharmaceutical composition for treating or preventing eye diseases, comprising the fusion protein or the fusion protein dimer as an active ingredient.

### Effects of Invention

The fusion protein comprising an antibody fragment that specifically binds to C3b or C5; and a protein that specifically binds to VEGF according to the present invention may not only efficiently inhibit complement-related mechanisms, but may also effectively inhibit angiogenesis. Therefore, eye diseases caused by the complement system and eye diseases caused by angiogenesis may be effectively treated or prevented. Therefore, the fusion protein may be usefully utilized to effectively treat macular degeneration, particularly both dry macular degeneration and wet macular degeneration.

### Brief Description of Drawings

Figures 1a to 1c illustrate results obtained by identifying the prepared MOR09611, MOR09675, S77, eculizumab, PRO236, PRO237, PRO238, PRO239, PRO240, PRO241, PRO242, PRO243, PRO017, KNP-301, and aflibercept by SDS-PAGE.

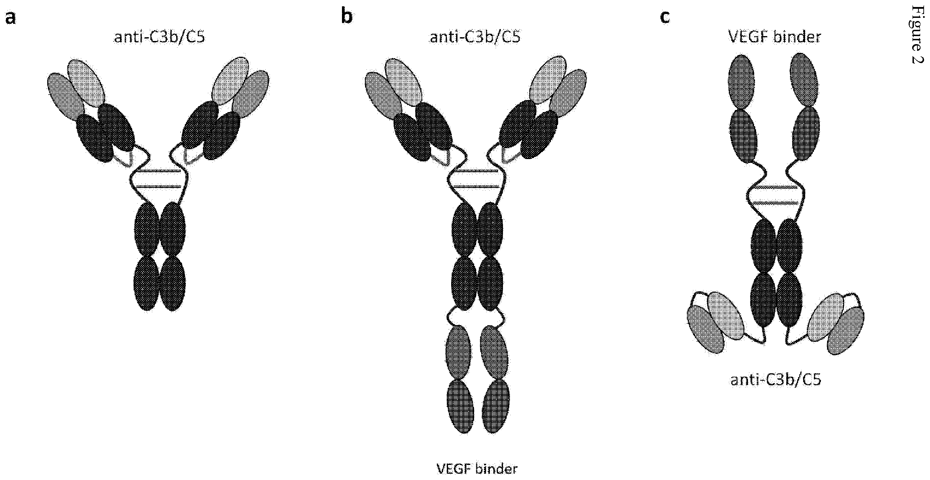

Figure 2 is a schematic diagram showing an example of the bispecific antibody of the present invention.
Figures 3a and 3b are graphs showing results obtained by measuring the binding affinity of KNP-301, PRO236, PRO237, PRO238, PRO239, PRO241, PRO242, S77, eculizumab, MOR09611, and MOR09675 to human C3b through ELISA.
Figure 4 is a graph showing a result obtained by measuring the binding affinity of PRO239, eculizumab, and PRO017 to human C5 through ELISA.
Figures 5a and 5b are graphs showing results obtained by measuring the binding affinity of aflibercept, PRO236, PRO237, PRO238, PRO017, PRO239, PRO241, and PRO242 to human VEGF165 through ELISA.
Figures 6a to 6c are graphs showing results obtained by measuring the alternative complement pathway inhibitory effect of KNP-301, PRO236, PRO237, PRO239, PRO241, PRO242, S77, eculizumab, MOR09611, and MOR09675 through hemolysis assay (AH50).
Figures 7a to 7c are graphs showing results obtained by measuring the classical complement pathway inhibitory effect of KNP-301, PRO236, PRO237, PRO239, PRO241, PRO242, S77, eculizumab, MOR09611, and MOR09675 through hemolysis assay (CH50).
Figures 8a to 8c are graphs showing results obtained by measuring the VEGF signaling inhibitory effect of aflibercept, PRO236, PRO237, PRO238, PRO239, PRO241, PRO242, and PRO017 using reporter cells.

### Best Mode for Carrying out the Invention

### Fusion protein comprising an antibody fragment that specifically binds to C3b or C5

In one aspect of the present invention, there is provided a fusion protein comprising an antibody fragment that specifically binds to C3b (complement component 3b) or C5 (complement component 5); and a protein that specifically binds to VEGF (vascular endothelial growth factor).

As used herein, the term "C3b (complement component 3b)" refers to a fragment of C3 formed by cleaving complement component C3 by C3 convertase, and may be used interchangeably with "complement C3b protein". The C3b particularly plays a central role in the complement system and binds to the surface of cells to act as a marker. In addition, it has an opsonization effect that allows phagocytic cells with C3b receptors to recognize it and cause cytotoxicity. In addition, it activates C5 to form a membrane attack complex (MAC) (kubi immunology, W. H. Freeman and Company, New York).

As used herein, the term "complement component", "complement protein," or "complement component protein" refers to molecules involved in activation of the complement system. Classical pathway components include, for example, the C1q, C1r, C1s, C2, C3, C4, C5, C6, C7, C8, C9, and C5b-9 complexes. Alternative pathway components include, for example, factor B, factor D, properdin, factor H, and factor I.

As used herein, the term "antibody fragment that specifically binds to C3b" may be used interchangeably with an "anti-C3b antibody", and refers to an antibody that specifically binds to the complement C3b protein or an antigen binding fragment thereof.

The antibody that specifically binds to C3b or an antigen binding fragment thereof specifically binds to the complement component C3b and blocks the activation of complement pathway. Accordingly, it may be used to treat disorders associated with the activation of complement pathway, such as macular degeneration-related disorders (including, for example, AMD, North Carolina macular dystrophy, Sorsby fundus dystrophy, Stargardt disease, pattern dystrophy, Best's disease, dominant drusen and Malattia Leventinese (radial drusen), extramacular changes occurring before or after macular degeneration and/or dysfunction, retinal detachment, chorioretinal degeneration, retinal degeneration, photoreceptor degeneration, RPE degeneration, mucopolysaccharidosis, rod-cone dystrophy, cone-rod dystrophy, and cone degeneration, etc.).

The "antibody that specifically binds to C3b" comprises at least two heavy chains and two light chains, interconnected by disulfide bonds. The heavy chain consists of a heavy chain variable region (VH) and a heavy chain constant region, and the light chain consists of a light chain variable region (VL) and a light chain constant region. The VH and VL regions may be further subdivided into hypervariable regions called complementarity determining regions (CDRs), interspersed with more conserved regions called framework regions (FRs).

The "antigen binding fragment of an antibody that specifically binds to C3b" refers to one or more fragments of an intact antibody that retains the ability to specifically bind to a given antigen, i.e., C3b. Examples of binding fragments included within the term "antigen-binding portion" of an antibody include Fab, scFv, F(ab')₂, diabody, triabody, sdAb, and V_{H}H.

In one embodiment, the antibody that specifically binds to C3b or an antigen binding fragment thereof may comprise a heavy chain variable region comprising HCDR1 of SEQ ID NO: 20, HCDR2 of SEQ ID NO: 21, and HCDR3 of SEQ ID NO: 22; and a light chain variable region comprising LCDR1 of SEQ ID NO: 23, LCDR2 of SEQ ID NO: 24, and LCDR3 of SEQ ID NO: 25. In addition, it may comprise a heavy chain variable region comprising HCDR1 of SEQ ID NO: 28, HCDR2 of SEQ ID NO: 29, and HCDR3 of SEQ ID NO: 30; and a light chain variable region comprising LCDR1 of SEQ ID NO: 31, LCDR2 of SEQ ID NO: 32, and LCDR3 of SEQ ID NO: 33. In addition, it may comprise a heavy chain variable region comprising HCDR1 of SEQ ID NO: 36, HCDR2 of SEQ ID NO: 37, and HCDR3 of SEQ ID NO: 38; and a light chain variable region comprising LCDR1 of SEQ ID NO: 39, LCDR2 of SEQ ID NO: 40, and LCDR3 of SEQ ID NO: 41.

In another embodiment, the antibody that specifically binds to C3b or an antigen binding fragment thereof may comprise a heavy chain variable region comprising the amino acid sequence of SEQ ID NO: 26 and a light chain variable region comprising the amino acid sequence of SEQ ID NO: 27. In addition, it may comprise a heavy chain variable region comprising the amino acid sequence of SEQ ID NO: 34 and a light chain variable region comprising the amino acid sequence of SEQ ID NO: 35. In addition, it may comprise a heavy chain variable region comprising the amino acid sequence of SEQ ID NO: 42 and a light chain variable region comprising the amino acid sequence of SEQ ID NO: 43.

The anti-C3b antibody may refer to any antibody known to those of ordinary skill in the art without limitation. As another example, the antibody may be an anti-C3b antibody or a fragment thereof disclosed in U.S. Patent Publication No. US 2010-0291106 A1 or U.S. Patent No. US 8,377,437 B2.

As used herein, the term "C5 (complement component 5)" may be used interchangeably with "complement C5 protein", and is a protein that is cleaved into C5a and C5b fragments by C5 convertase and released. C5a is an anaphylatoxin that releases neutrophils and inflammation-mediated cytokines, and C5b combines with C6, C7, C8, and C9 to form a membrane attack complex, thereby destroying cell membranes. When the concentration of C5a in the body is maintained high, the expressions of IL-17 and IL-22 are increased, and the increased expressions of IL-17 and IL-22 are known to act as an inflammatory factors and induce VEGF and angiogenesis (Liu B. et.al., J. Transl. Med., 2011;9:1-12).

As used herein, the term "antibody fragment that specifically binds to C5" may be used interchangeably with an "anti-C5 antibody", and refers to an antibody that specifically binds to the complement C5 protein or an antigen binding fragment thereof.

The antibody that specifically binds to C5 or an antigen binding fragment thereof specifically binds to the complement component C5 and blocks the activation of complement pathway. Accordingly, it may be used to treat disorders associated with the activation of complement pathway, such as macular degeneration-related disorders.

The "antibody that specifically binds to C5" comprises at least two heavy chains and two light chains, interconnected by disulfide bonds. The heavy chain and the light chain are as described above.

The "antigen binding fragment of an antibody that specifically binds to C5" refers to one or more fragments of an intact antibody that retains the ability to specifically bind to a given antigen, i.e., C5. Examples of binding fragments included within the term "antigen-binding portion" of an antibody include Fab, scFv, F(ab')₂, diabody, triabody, sdAb, and V_{H}H.

In one embodiment, the antibody that specifically binds to C5 or an antigen binding fragment thereof may comprise a heavy chain variable region comprising HCDR1 of SEQ ID NO: 44, HCDR2 of SEQ ID NO: 45, and HCDR3 of SEQ ID NO: 46; and a light chain variable region comprising LCDR1 of SEQ ID NO: 47, LCDR2 of SEQ ID NO: 48, and LCDR3 of SEQ ID NO: 49.

In another embodiment, the antibody that specifically binds to C5 or an antigen binding fragment thereof may comprise a heavy chain variable region comprising the amino acid sequence of SEQ ID NO: 50 and a light chain variable region comprising the amino acid sequence of SEQ ID NO: 51.

The anti-C5 antibody may refer to any antibody known to those of ordinary skill in the art without limitation. As another example, the antibody may be an anti-C5 antibody or a fragment thereof disclosed in U.S. Patent No. US 6,355,245 B2 or U.S. Patent Publication No. US 2019-0177436 A1.

As used herein, the term "VEGF (vascular endothelial growth factor)" is referred to as vascular endothelial growth factor, and causes angiogenesis by binding to its receptor, VEGFR (VEGF receptor). VEGF plays a role in inducing endothelial cell proliferation, migration, and differentiation by activating various signaling cascades. Under pathological conditions, VEGF induces abnormal angiogenesis and promotes the growth of tumor cells and retinal cells and vascular leakage, thereby causing tumor growth and metastasis, diabetic retinopathy, and age-related macular degeneration, etc.

In addition, in the present specification, VEGF or VEGF family proteins are collectively expressed as the term "VEGF". The VEGF family proteins may have equivalent or similar activities to VEGF. At this time, "activity" may mean, for example, specific binding to the VEGF receptor, and this specific binding may be measured through methods known to those of ordinary skill in the art.

The VEGF family protein may be one or more selected from the group consisting of VEGF-A, VEGF-B, VEGF-C, VEGF-D, VEGF-E, PIGF (placental growth factor), and recombinant VEGF. Preferably, the VEGF may be VEGF-A, VEGF-B, or PIGF, which are preferentially required for angiogenesis.

As used herein, the term "PIGF (placental growth factor)" refers to a transmembrane protein encoded by chromosome 2p21-p16. The PlGF acts as a selective ligand for VEGFR-1 and may promote angiogenesis. The PIGF has an amino acid composition that is at least 40% identical to that of VEGF. In one embodiment, the PIGF may be PlGF-1 or PIGF-2.

As used herein, the term "recombinant VEGF" refers to VEGF recombined through alternative exon splicing. The recombinant VEGF may be, for example, VEGF111, VEGF121, VEGF145, VEGF148, VEFG165, VEGF183, VEGF189, or VEGF206 depending on the number of amino acids, but is not limited thereto.

As used herein, the term "protein that specifically binds to VEGF" refers to an antibody that specifically binds to VEGF or the extracellular domain of the VEGF receptor.

As used herein, the term "VEGF receptor" refers to a receptor that binds to VEGF, and the biological functions of VEGF are mediated through VEGFR-1 (Flt-1), VEGFR-2 (KDR/Flk-1), and VEGFR-3 (Flt-4). VEGFR consists of seven extracellular immunoglobulin (Ig)-like domains, a transmembrane (TM) domain, a regulatory juxtamembrane domain, an intracellular tyrosine kinase domain, and several other tyrosine residues. Specifically, the extracellular immunoglobulin (Ig)-like domain of VEGFR-1 and VEGFR-2 is known to be a domain that binds to VEGF.

In the present invention, the VEGF receptor may be VEGF receptor 1 (VEGFR-1) or VEGF receptor 2 (VEGFR-2), but is not limited thereto.

As used herein, the term "extracellular domain of the VEGF receptor" refers to the domain of the VEGF receptor that binds to the VEGF. Specifically, it refers to the extracellular domain portion comprising the ligand of VEGF, excluding the transmembrane region and cytoplasmic region of the VEGF receptor.

Specifically, the extracellular domain of the VEGF receptor may be a fragment of the VEGF receptor that binds to VEGF. In addition, the extracellular domain of the VEGF receptor may inhibit angiogenesis by binding to VEGF-A, VEGF-B, or PIGF. At this time, one embodiment of the extracellular domain of the VEGF receptor may comprise the amino acid sequence of SEQ ID NO: 52. In addition, the extracellular domain of the VEGF receptor may be a truncated or altered form of a portion of the extracellular domain of the VEGF receptor comprising SEQ ID NO: 52.

As used herein, the term "antibody that specifically binds to VEGF" refers to an antibody that specifically binds to VEGF and causes an antigen-antibody reaction or a fragment thereof, and is also referred to as an anti-VEGF antibody.

The antibody is a general term for molecules capable of specific antigen-antibody binding to VEGF. In addition, the antibody may be used in any form as long as it comprises an antigen binding domain capable of specifically binding to VEGF. The antibody or a fragment thereof may be Fab (fragment antigen binding), F(ab)₂, scFv (single-chain variable fragment), di-scFv, sdAb (single domain antibody), chimeric antibody, humanized-antibody, human antibody or a variant thereof.

The anti-VEGF antibody may comprise a variable site of any one selected from the group consisting of aflibercept, bevacizumab, ranibizumab, ramucirumab, brolucizumab, faricimab, KSI-301, vanucizumab, BI-836880, HuMab G6-31, B20-4.1, BAT-5906, navicixizumab, dilpacimab, hPV-19, and AT-001. Preferably, the anti-VEGF antibody may comprise a variable region of aflibercept, bevacizumab, ranibizumab, brolucizumab, KSI-301, vanucizumab, BI-836880, or BAT-5906.

At this time, the aflibercept refers to a recombinant humanized fusion protein that inhibits VEGF-A and PlGF in blood vessels. The aflibercept may be directly injected into the eye. The bevacizumab is an antibody that is an angiogenesis inhibitor that inhibits the growth of blood vessels by inhibiting VEGF-A within blood vessels. For the treatment of macular degeneration, the bevacizumab may be directly injected into the eye. The ranibizumab is a Fab that is effective in treating wet macular degeneration by inhibiting angiogenesis. The ramucirumab is a substance that mediates angiogenesis or an antibody that inhibits VEGF receptor 2. The brolucizumab is an scFv that binds to VEGF-A, inhibits angiogenesis, and treats wet macular degeneration. The faricimab is a bispecific antibody that inhibits VEGF-A and angiopoietin-2.

In addition, the KSI-301 is an antibody effective in treating wet macular degeneration. The vanucizumab is a bispecific humanized monoclonal antibody that inhibits VEGF-A and angiopoietin-2. The BI-836880 is a humanized bispecific nanobody that inhibits VEGF and angiopoietin-2. The HuMab G6-31 is a Fab fragment that inhibits human VEGF. The B20-4.1 is an scFv fragment that inhibits human VEGF.

In addition, the BAT-5906 is an antibody effective in treating wet macular degeneration. The navicixizumab is an anti-DLL4/VEGF bispecific antibody. The dilpacimab is an anti-DLL4/VEGF bispecific antibody and is also referred to as ABT-165. The hPV-19 is an anti-VEGF antibody having anti-angiogenic and anti-tumor activities. The AT-001 is an antibody that inhibits angiogenesis by inhibiting human VEGF receptor 3.

In one embodiment, the anti-VEGF antibody may comprise a variable region of BI-836880. Specifically, the antibody may comprise a heavy chain variable region comprising CDR1 of SEQ ID NO: 88, CDR2 of SEQ ID NO: 89, and CDR3 of SEQ ID NO: 90.

In one embodiment, the anti-VEGF antibody may comprise a variable region of bevacizumab. Specifically, the antibody may comprise a heavy chain variable region comprising HCDR1 of SEQ ID NO: 91, HCDR2 of SEQ ID NO: 92, and HCDR3 of SEQ ID NO: 93; and a light chain variable region comprising LCDR1 of SEQ ID NO: 94, LCDR2 of SEQ ID NO: 95, and LCDR3 of SEQ ID NO: 96.

In one embodiment, the anti-VEGF antibody may comprise a variable region of ranibizumab. Specifically, the antibody may comprise a heavy chain variable region comprising HCDR1 of SEQ ID NO: 97, HCDR2 of SEQ ID NO: 98, and HCDR3 of SEQ ID NO: 99; and a light chain variable region comprising LCDR1 of SEQ ID NO: 100, LCDR2 of SEQ ID NO: 101, and LCDR3 of SEQ ID NO: 102.

In one embodiment, the anti-VEGF antibody may comprise a variable region of ramucirumab. Specifically, the antibody may comprise a heavy chain variable region comprising HCDR1 of SEQ ID NO: 103, HCDR2 of SEQ ID NO: 104, and HCDR3 of SEQ ID NO: 105; and a light chain variable region comprising LCDR1 of SEQ ID NO: 106, LCDR2 of SEQ ID NO: 107, and LCDR3 of SEQ ID NO: 108.

In one embodiment, the anti-VEGF antibody may comprise a variable region of faricimab. Specifically, the antibody may comprise a heavy chain variable region comprising HCDR1 of SEQ ID NO: 109, HCDR2 of SEQ ID NO: 110, and HCDR3 of SEQ ID NO: 111; and a light chain variable region comprising LCDR1 of SEQ ID NO: 112, LCDR2 of SEQ ID NO: 113, and LCDR3 of SEQ ID NO: 114.

In one embodiment, the anti-VEGF antibody may comprise a variable region of KSI-301. Specifically, the antibody may comprise a heavy chain variable region comprising HCDR1 of SEQ ID NO: 115, HCDR2 of SEQ ID NO: 116, and HCDR3 of SEQ ID NO: 117; and a light chain variable region comprising LCDR1 of SEQ ID NO: 118, LCDR2 of SEQ ID NO: 119, and LCDR3 of SEQ ID NO: 120.

In one embodiment, the anti-VEGF antibody may comprise a variable region of vanucizumab. Specifically, the antibody may comprise a heavy chain variable region comprising HCDR1 of SEQ ID NO: 121, HCDR2 of SEQ ID NO: 122, and HCDR3 of SEQ ID NO: 123; and a light chain variable region comprising LCDR1 of SEQ ID NO: 124, LCDR2 of SEQ ID NO: 125, and LCDR3 of SEQ ID NO: 126.

In one embodiment, the anti-VEGF antibody may comprise a variable region of BAT-5906. Specifically, the antibody may comprise a heavy chain variable region comprising HCDR1 of SEQ ID NO: 127, HCDR2 of SEQ ID NO: 128, and HCDR3 of SEQ ID NO: 129; and a light chain variable region comprising LCDR1 of SEQ ID NO: 130, LCDR2 of SEQ ID NO: 131, and LCDR3 of SEQ ID NO: 132.

In one embodiment, the anti-VEGF antibody may comprise a variable region of navicixizumab. Specifically, the antibody may comprise a heavy chain variable region comprising HCDR1 of SEQ ID NO: 133, HCDR2 of SEQ ID NO: 134, and HCDR3 of SEQ ID NO: 135; and a light chain variable region comprising LCDR1 of SEQ ID NO: 136, LCDR2 of SEQ ID NO: 137, and LCDR3 of SEQ ID NO: 138.

In one embodiment, the anti-VEGF antibody may comprise a variable region of dilpacimab. Specifically, the antibody may comprise a heavy chain variable region comprising HCDR1 of SEQ ID NO: 139, HCDR2 of SEQ ID NO: 140, and HCDR3 of SEQ ID NO: 141; and a light chain variable region comprising LCDR1 of SEQ ID NO: 142, LCDR2 of SEQ ID NO: 143, and LCDR3 of SEQ ID NO: 144.

In one embodiment, the anti-VEGF antibody may comprise a variable region of hPV-19. Specifically, the antibody may comprise a heavy chain variable region comprising HCDR1 of SEQ ID NO: 145, HCDR2 of SEQ ID NO: 146, and HCDR3 of SEQ ID NO: 147; and a light chain variable region comprising LCDR1 of SEQ ID NO: 148, LCDR2 of SEQ ID NO: 149, and LCDR3 of SEQ ID NO: 150.

In one embodiment, the anti-VEGF antibody may comprise a variable region of AT-001. Specifically, the antibody may comprise a heavy chain variable region comprising HCDR1 of SEQ ID NO: 151, HCDR2 of SEQ ID NO: 152, and HCDR3 of SEQ ID NO: 153; and a light chain variable region comprising LCDR1 of SEQ ID NO: 154, LCDR2 of SEQ ID NO: 155, and LCDR3 of SEQ ID NO: 156.

In one embodiment, the anti-VEGF antibody may comprise the heavy chain of SEQ ID NO: 157 and the light chain of SEQ ID NO: 158; the heavy chain variable region of SEQ ID NO: 159 and the light chain of SEQ ID NO: 160; the heavy chain of SEQ ID NO: 161 and the light chain of SEQ ID NO: 162; the heavy chain of SEQ ID NO: 163 and the light chain of SEQ ID NO: 164; the heavy chain of SEQ ID NO: 165 and the light chain of SEQ ID NO: 166; the heavy chain of SEQ ID NO: 167 and the light chain of SEQ ID NO: 168; the heavy chain of SEQ ID NO: 169 and the light chain of SEQ ID NO: 170; the heavy chain of SEQ ID NO: 171 and the light chain of SEQ ID NO: 172; the heavy chain of SEQ ID NO: 173 and the light chain of SEQ ID NO: 174; the heavy chain variable region of SEQ ID NO: 175 and the light chain variable region of SEQ ID NO: 176; or the heavy chain variable region of SEQ ID NO: 177 and the light chain variable region of SEQ ID NO: 178.

The fragment of the anti-VEGF antibody may be a scFv (single chain variable fragment). At this time, the scFv refers to a form in which a heavy chain variable region and a light chain variable region are linked by a peptide linker. Specifically, the scFv may comprise a variable region comprising CDR1 of SEQ ID NO: 179, CDR2 of SEQ ID NO: 180, CDR3 of SEQ ID NO: 181, CDR4 of SEQ ID NO: 182, CDR5 of SEQ ID NO: 183, and CDR6 of SEQ ID NO: 184. In addition, the scFv may have the amino acid sequence of SEQ ID NO: 185. At this time, one embodiment of the scFv may be brolucizumab.

The anti-VEGF antibody may comprise a variable region of HuMab G6-31 or B20-4.1. Specifically, the antibody may comprise a heavy chain variable region comprising HCDR1 of SEQ ID NO: 186, HCDR2 of SEQ ID NO: 187, and HCDR3 of SEQ ID NO: 188; and a light chain variable region comprising LCDR1 of SEQ ID NO: 189, LCDR2 of SEQ ID NO: 190, and LCDR3 of SEQ ID NO: 191. In addition, the antibody may comprise a heavy chain variable region comprising HCDR1 of SEQ ID NO: 192, HCDR2 of SEQ ID NO: 193, and HCDR3 of SEQ ID NO: 194; and a light chain variable region comprising LCDR1 of SEQ ID NO: 195, LCDR2 of SEQ ID NO: 196, and LCDR3 of SEQ ID NO: 197.

The antibody that specifically binds to VEGF may refer to any antibody known to those of ordinary skill in the art without limitation. As another example, the antibody may be an anti-VEGF antibody or a fragment thereof disclosed in U.S. Patent No. US 9,527,925 B2, U.S. Patent No. US 8,268,314 B2 or U.S. Patent Publication No. US 2019-0167790 A1.

### Linker and Fc variant

The antibody fragment that specifically binds to C3b or C5; and the protein that specifically binds to VEGF may be linked via a linker. At this time, the linker may be a peptide linker, an immunoglobulin fragment, or a combination thereof, but is not limited thereto.

The linker links two proteins. One embodiment of the linker may include 1 to 50 amino acids, albumin or a fragment thereof, an Fc domain of an immunoglobulin, or the like. At this time, the Fc domain of an immunoglobulin refers to a protein that comprises heavy chain constant region 2 (CH2) and heavy chain constant region 3 (CH3) of an immunoglobulin, and does not comprise heavy and light chain variable regions and light chain constant region 1 (CH1) of an immunoglobulin. The immunoglobulin may be IgG, IgA, IgE, IgD, or IgM, and may preferably be IgG1. At this time, an Fc domain of wild type immunoglobulin G1 may have the amino acid sequence of SEQ ID NO: 68. As used herein, the Fc domain may refer to a region including CH2 and CH3 domains, excluding a hinge region.

In addition, the Fc domain of an immunoglobulin may be an Fc domain variant as well as a wild type Fc domain. In addition, as used herein, the term "Fc domain variant" may refer to a form which is different from the wild type Fc domain in terms of glycosylation pattern, has a high glycosylation as compared with the wild type Fc domain, or has a low glycosylation as compared with the wild type Fc domain, or a deglycosylated form. In addition, an aglycosylated Fc domain is included therein. The Fc domain or a variant thereof may be adapted to have an adjusted number of sialic acids, fucosylations, or glycosylations, through culture conditions or genetic manipulation of a host.

In addition, glycosylation of the Fc domain of an immunoglobulin may be modified by conventional methods such as chemical methods, enzymatic methods, and genetic engineering methods using microorganisms. In addition, the Fc domain variant may be in a mixed form of respective Fc regions of immunoglobulin IgG, IgA, IgE, IgD, or IgM. In addition, the Fc domain variant may be in a form in which some amino acids of the Fc domain are substituted with other amino acids.

As used herein, the term "Fc domain variant" refers to an Fc domain in which the glycosylation of the wild type Fc domain is altered, sequences between Fc domains are mixed, or some amino acids of the wild type Fc domain are deleted, altered, substituted, and/or added. Deletion, alteration, substitution, and/or addition of some amino acids of the wild type Fc domain may be made by methods known to those of ordinary skill in the art. In one embodiment, the Fc domain variant may be one in which some amino acid sequences of the wild type Fc domain are substituted and/or added.

The "amino acid" introduced by the substitution and/or addition may be any one selected from the group consisting of lysine (K), alanine (A), arginine (R), asparagine (N), aspartic acid (D), cysteine (C), glutamine (Q), glutamic acid (E), glycine (G), histidine (H), isoleucine (I), leucine (L), methionine (M), phenylalanine (F), proline (P), serine (S), threonine (T), tryptophan (W), tyrosine (Y), and valine (V).

The Fc domain mutation may regulate the activity or function of the antibody. In one embodiment, the Fc domain mutation may regulate the effector function or antibody cytotoxic activities of the antibody.

In one embodiment, the Fc domain variant may include a DANG mutation. At this time, a "DANG mutation" refers to the D265A/N297G mutation for removing an effector function in human IgG1 or mouse IgG2a.

The effector functions mediated by the Fc region in an IgG molecule include C1q binding, complement dependent cytotoxicity, Fc receptor binding, antibody dependent cell mediated cytotoxicity (ADCC), phagocytosis, downregulation of cell surface receptors (for example, B cell receptor, BCR) and the like. In general, these effector functions need to the binding of the Fc region with a binding domain (for example, an antibody variable domain).

The effector function may be changed by the substitution of the amino acid sequence of the non-mutated Fc region, and the Fc region in which the effector function is changed may be designed for example, by modifying C1q binding and/or FcR binding, thereby changing CDC activity and/or ADCC activity. That is, the "DANG mutation" means that the effector function mediated by the Fc region is removed from the IgG molecule so that an unwanted effector fuction does not occur during antibody production.

One embodiment of the Fc domain variant may be a substitution of D27A, N59G, D118E, L120M, D27A/N59G, or D27A/N59G/D118E/L120M in the amino acid sequence of SEQ ID NO: 68. In addition, lysine (K) may be added to the 209th position in the amino acid sequence of SEQ ID NO: 68. Specifically, one embodiment of the Fc domain variant may have any one of the amino acid sequences of SEQ ID NOs: 62 to 67.

The fusion protein may have a structure in which, using an Fc domain as a linker, an antibody fragment that specifically binds to C3b or C5 and a protein that specifically binds to VEGF are linked, or a protein that specifically binds to VEGF and an antibody fragment that specifically binds to C3b or C5 are linked to the N-terminus and C-terminus thereof, respectively. Linkage between the N-terminus or C-terminus of the Fc domain and an antibody fragment that specifically binds to C3b or C5 or a protein that specifically binds to VEGF may optionally be achieved by a linker peptide.

### Structure of fusion protein

Specifically, the fusion protein may consist of the following structural formula (I) or (II):

N'-X-[linker (1)]n-Fc domain or variant thereof-[linker (2)]m-Y-C' (I)

N'-Y-[linker (1)]n-Fc domain or variant thereof-[linker (2)]m-X-C' (II)

in the structural formulas (I) and (II),
N' is the N-terminus of the fusion protein,
C' is the C-terminus of the fusion protein,
X is the antibody fragment that specifically binds to C3b or C5,
Y is the protein that specifically binds to VEGF,
the linkers (1) and (2) are peptide linkers, and
n and m are each independently 0 or 1.

The antibody fragment that specifically binds to C3b or C5, the protein that specifically binds to VEGF, and the Fc domain are each as described above.

As used herein, the term "fusion protein" refers to a recombinant protein in which two or more proteins or domains responsible for a specific function within a protein are linked so that each protein or domain performs its own function. A linker peptide, which typically has a flexible structure, may be inserted between the two or more proteins or domains.

At this time, the peptide linker (1) may consist of 5 to 80 consecutive amino acids, 7 to 70 consecutive amino acids, or 10 to 60 consecutive amino acids, or 12 to 50 amino acids. In one embodiment, the peptide linker (1) may consist of 30 amino acids. In addition, the peptide linker (1) may comprise at least one cysteine. Specifically, it may comprise 1, 2, or 3 cysteines. In addition, the peptide linker (1) may be derived from the hinge of immunoglobulin, and may further comprise (G₄S)n (where n is an integer of 1 to 10). In one embodiment, the peptide linker (1) may be a peptide linker consisting of any one of the amino acid sequences of SEQ ID NOs: 53 to 57.

The peptide linker (2) may consist of 1 to 50 consecutive amino acids, or 3 to 30 consecutive amino acids, or 5 to 20 amino acids. In one embodiment, the peptide linker (2) may be (G₄S)n (where n is an integer of 1 to 10). At this time, in (G₄S)n, n may be 1, 2, 3, 4, 5, 6, 7, 8, 9, or 10. In one embodiment, the peptide linker (2) may be a peptide linker consisting of any one of the amino acid sequences of SEQ ID NOs: 58 to 61.

In one embodiment, the antibody fragment may be Fab or scFv that specifically binds to C3b.

Specifically, the Fab or scFv that specifically binds to C3b may comprise a heavy chain variable region comprising HCDR1 of SEQ ID NO: 20, HCDR2 of SEQ ID NO: 21, and HCDR3 of SEQ ID NO: 22; and a light chain variable region comprising LCDR1 of SEQ ID NO: 23, LCDR2 of SEQ ID NO: 24, and LCDR3 of SEQ ID NO: 25.

In addition, the Fab or scFv that specifically binds to C3b may comprise a heavy chain variable region comprising HCDR1 of SEQ ID NO: 28, HCDR2 of SEQ ID NO: 29, and HCDR3 of SEQ ID NO: 30; and a light chain variable region comprising LCDR1 of SEQ ID NO: 31, LCDR2 of SEQ ID NO: 32, and LCDR3 of SEQ ID NO: 33.

In addition, the Fab or scFv that specifically binds to C3b may comprise a heavy chain variable region comprising HCDR1 of SEQ ID NO: 36, HCDR2 of SEQ ID NO: 37, and HCDR3 of SEQ ID NO: 38; and a light chain variable region comprising LCDR1 of SEQ ID NO: 39, LCDR2 of SEQ ID NO: 40, and LCDR3 of SEQ ID NO: 41.

In one embodiment, the Fab or scFv that specifically binds to C3b may comprise a heavy chain variable region comprising the amino acid sequence of SEQ ID NO: 26 and a light chain variable region comprising the amino acid sequence of SEQ ID NO: 27. In addition, the Fab or scFv that specifically binds to C3b may comprise a heavy chain variable region comprising the amino acid sequence of SEQ ID NO: 34 and a light chain variable region comprising the amino acid sequence of SEQ ID NO: 35. In addition, the Fab or scFv that specifically binds to C3b may comprise a heavy chain variable region comprising the amino acid sequence of SEQ ID NO: 42 and a light chain variable region comprising the amino acid sequence of SEQ ID NO: 43.

In one embodiment, the antibody fragment may be Fab or scFv that specifically binds to C5.

Specifically, the Fab or scFv that specifically binds to C5 may comprise a heavy chain variable region comprising HCDR1 of SEQ ID NO: 44, HCDR2 of SEQ ID NO: 45, and HCDR3 of SEQ ID NO: 46; and a light chain variable region comprising LCDR1 of SEQ ID NO: 47, LCDR2 of SEQ ID NO: 48, and LCDR3 of SEQ ID NO: 49.

In one embodiment, the Fab or scFv that specifically binds to C5 may comprise a heavy chain variable region comprising the amino acid sequence of SEQ ID NO: 50 and a light chain variable region comprising the amino acid sequence of SEQ ID NO: 51.

The fusion protein of the present invention may comprise a fusion protein comprising a light chain variable region and a light chain constant region of an anti-C3b antibody, and a heavy chain variable region and a heavy chain constant region (CH1) of an anti-C3b antibody, an Fc domain, and a protein that specifically binds to VEGF. At this time, the at least one protein that specifically binds to VEGF may be included in an anti-C3b antibody. In addition, the protein that specifically binds to VEGF may be bound to the C terminus of an anti-C3b antibody.

In addition, the fusion protein of the present invention may comprise a fusion protein comprising a light chain variable region and a light chain constant region of an anti-C5 antibody, and a heavy chain variable region and a heavy chain constant region (CH1) of an anti-C5 antibody, an Fc domain, and a protein that specifically binds to VEGF. At this time, the at least one protein that specifically binds to VEGF may be included in an anti-C5 antibody. In addition, the protein that specifically binds to VEGF may be bound to the C terminus of an anti-C5 antibody.

In one embodiment, the fusion protein may comprise the polypeptide of SEQ ID NO: 2 and the polypeptide of SEQ ID NO: 9; the polypeptide of SEQ ID NO: 4 and the polypeptide of SEQ ID NO: 10; the polypeptide of SEQ ID NO: 6 and the polypeptide of SEQ ID NO: 11; the polypeptide of SEQ ID NO: 8 and the polypeptide of SEQ ID NO: 12; the polypeptide of SEQ ID NO: 13; the polypeptide of SEQ ID NO: 14; the polypeptide of SEQ ID NO: 15; or the polypeptide of SEQ ID NO: 16.

Specifically, SEQ ID NOs: 2, 4, and 6 are the amino acid sequences of a fusion protein comprising a light chain variable region and a light chain constant region of an anti-C3b antibody, respectively. In addition, SEQ ID NOs: 9, 10, and 11 are amino acid sequences comprising i) a heavy chain variable region and a heavy chain constant region (CH1) of an anti-C3b antibody, ii) an Fc domain, and iii) a protein that specifically binds to VEGF, respectively.

In addition, SEQ ID NO: 8 is the amino acid sequence of a fusion protein comprising a light chain variable region and a light chain constant region of an anti-C5 antibody, and SEQ ID NO: 12 is the amino acid sequence comprising i) a heavy chain variable region and a heavy chain constant region (CH1) of an anti-C5 antibody, ii) an Fc domain, and iii) a protein that specifically binds to VEGF.

At this time, the combination of SEQ ID NOs: 2 and 9, the combination of SEQ ID NOs: 4 and 10, the combination of SEQ ID NOs: 6 and 11, and the combination of SEQ ID NOs: 8 and 12 are each linked by a disulfide (-S-S-) bond and are maintained together by non-covalent interaction.

Specifically, SEQ ID NOs: 13, 14, and 15 are the amino acid sequences comprising an scFv comprising i) a protein that specifically binds to VEGF, ii) an Fc domain, iii) a heavy chain variable region of an anti-C3b antibody, and iv) a light chain variable region of an anti-C3b antibody, respectively.

In addition, SEQ ID NO: 16 is the amino acid sequence comprising an scFv comprising i) a protein that specifically binds to VEGF, ii) an Fc domain, iii) a heavy chain variable region of an anti-C5 antibody, and iv) a light chain variable region of an anti-C5 antibody.

In another embodiment, the fusion protein includes a polypeptide having a sequence identity of 85%, 86%, 87%, 88%, 89%, 90%, 91%, 92%, 93%, 94%, 95%, 96%, 97%, 98%, 99%, or 100% to the polypeptide of SEQ ID NO: 2 and the polypeptide of SEQ ID NO: 9; the polypeptide of SEQ ID NO: 4 and the polypeptide of SEQ ID NO: 10; the polypeptide of SEQ ID NO: 6 and the polypeptide of SEQ ID NO: 11; the polypeptide of SEQ ID NO: 8 and the polypeptide of SEQ ID NO: 12; the polypeptide of SEQ ID NO: 13; the polypeptide of SEQ ID NO: 14; the polypeptide of SEQ ID NO: 15; or the polypeptide of SEQ ID NO: 16. At this time, the identity is, for example, percent homology, and may be determined through homology comparison software such as BlastN software of the National Center of Biotechnology Information (NCBI).

### Fusion protein dimer

In another aspect of the present invention, there is provided a fusion protein dimer in which the two fusion proteins, which comprise an antibody fragment that specifically binds to C3b or C5 and a protein that specifically binds to VEGF, are attached to each other.

At this time, the binding between the fusion proteins constituting the dimer may be achieved by, but is not limited to, a disulfide bond formed by cysteines present in the linker. The fusion proteins constituting the dimer may be the same or different fusion proteins from each other. Preferably, the dimer may be a homodimer.

### Polynucleotide encoding a fusion protein

In another aspect of the present invention, there is provided a polynucleotide encoding a fusion protein comprising an antibody fragment that specifically binds to C3b or C5 and a protein that specifically binds to VEGF. Specifically, the polynucleotide may comprise the polynucleotide of SEQ ID NO: 77 and the polynucleotide of SEQ ID NO: 70; the polynucleotide of SEQ ID NO: 78 and the polynucleotide of SEQ ID NO: 72; the polynucleotide of SEQ ID NO: 79 and the polynucleotide of SEQ ID NO: 74; the polynucleotide of SEQ ID NO: 80 and the polynucleotide of SEQ ID NO: 76; the polynucleotide of SEQ ID NO: 81; the polynucleotide of SEQ ID NO: 82; the polynucleotide of SEQ ID NO: 83; or the polynucleotide of SEQ ID NO: 84. In the polynucleotide, one or more nucleotides may be mutated by substitution, deletion, insertion, or a combination thereof. When a nucleotide sequence is prepared by chemical synthesis, synthetic methods well known in the art may be used, such as those described in Engels and Uhlmann (Angew Chem IntEd Engl., 37:73-127, 1988). Such methods may include triester, phosphite, phosphoramidite and H-phosphate methods, PCR and other autoprimer methods, oligonucleotide syntheses on solid supports, and the like.

According to one embodiment, the polynucleotide may comprise a nucleic acid sequence having an identity of at least about 70%, at least about 75%, at least about 80%, at least about 85%, at least about 86%, at least about 87%, at least about 88%, at least about 89%, at least about 90%, at least about 91%, at least about 92%, at least about 93%, at least about 94%, at least about 95%, at least about 96%, at least about 97%, at least about 98%, at least about 99%, or at least about 100% to the polynucleotide of SEQ ID NO: 77 and the polynucleotide of SEQ ID NO: 70; the polynucleotide of SEQ ID NO: 78 and the polynucleotide of SEQ ID NO: 72; the polynucleotide of SEQ ID NO: 79 and the polynucleotide of SEQ ID NO: 74; the polynucleotide of SEQ ID NO: 80 and the polynucleotide of SEQ ID NO: 76; the polynucleotide of SEQ ID NO: 81; the polynucleotide of SEQ ID NO: 82; the polynucleotide of SEQ ID NO: 83; or the polynucleotide of SEQ ID NO: 84.

The polynucleotide may further comprise a nucleic acid encoding a signal sequence or a leader sequence. As used herein, the term "signal sequence" refers to a signal peptide that directs secretion of a target protein. The signal peptide is translated and then cleaved in a host cell. Specifically, the signal sequence is an amino acid sequence that initiates migration of a protein across the endoplasmic reticulum (ER) membrane.

Signal sequences are well known in the art for their characteristics. Such signal sequences typically comprise 16 to 30 amino acid residues, and may comprise more or fewer amino acid residues than such amino acid residues. Atypical signal peptide is composed of three regions, that is, a N-terminal region, a central hydrophobic region, and a more polar C-terminal region. The central hydrophobic region comprises 4 to 12 hydrophobic residues that cause the signal sequence to be immobilized during migration of an immature polypeptide through the membrane lipid bilayer.

After initiation, signal sequences are cleaved in the lumen of ER by cellular enzymes, commonly known as signal peptidases. At this time, the signal sequence may be a secretory signal sequence of tPa (tissue plasminogen activator), HSV gDs (signal sequence of Herpes simplex virus glycoprotein D), or a growth hormone. Preferably, a secretory signal sequence used in higher eukaryotic cells including mammals and the like may be used.

### Vector loaded with a polynucleotide encoding a fusion protein

In another aspect of the present invention, there is provided a vector comprising the polynucleotide.

The vector may be introduced into a host cell to be recombined with and inserted into the genome of the host cell. Alternatively, the vector is understood as nucleic acid means comprising a polynucleotide sequence which is autonomously replicable as an episome. The vectors include linear nucleic acids, plasmids, phagemids, cosmids, RNA vectors, viral vectors, and analogs thereof. Examples of the viral vector include, but are not limited to, retroviruses, adenoviruses, and adeno-associated viruses.

Specifically, the vector may include plasmid DNA, phage DNA, and the like; and commercially developed plasmids (pUC18, pBAD, pIDTSAMRT-AMP, and the like), E. coli-derived plasmids (pYG601BR322, pBR325, pUC118, pUC119, and the like), Bacillus subtilis-derived plasmids (pUB 110, pTP5, and the like), yeast-derived plasmids (YEp13, YEp24, YCp50, and the like), phage DNA (Charon4A, Charon21A, EMBL3, EMBL4, λgt10, λgt11, λZAP, and the like), animal viral vectors (retroviruses, adenoviruses, vaccinia viruses, and the like), insect viral vectors (baculoviruses and the like). Since the vector exhibits different expression levels and modification of a protein depending on a host cell, it is preferred to select and use a host cell which is most suitable for the purpose.

As used herein, the term "gene expression" or "expression" of a target protein is understood to mean transcription of DNA sequences, translation of mRNA transcripts, and secretion of fusion protein products or fragments thereof. A useful expression vector may be RcCMV (Invitrogen, Carlsbad) or a variant thereof. The expression vector may comprise human cytomegalovirus (CMV) promoter for promoting continuous transcription of a target gene in mammalian cells, and a bovine growth hormone polyadenylation signal sequence for increasing the stability level of RNA after transcription.

### Transformed cell expressing a fusion protein

In another aspect of the present invention, there is provided a transformed host cell into which the vector has been introduced.

Host cells for the transformed cell may include, but are not limited to, prokaryotic cells, eukaryotic cells, and cells of mammalian, plant, insect, fungal, or cellular origin. As an example of the prokaryotic cells, *E. coli* may be used. In addition, as an example of the eukaryotic cells, yeast may be used. In addition, for the mammalian cells, CHO cells, F2N cells, CSO cells, BHK cells, Bowes melanoma cells, HeLa cells, 911 cells, AT1080 cells, A549 cells, HEK 293 cells, HEK293T cells, or the like may be used. However, the mammalian cells are not limited thereto, and any cells which are known to those of ordinary skill in the art to be usable as mammalian host cells may be used.

In addition, for the introduction of an expression vector into the host cell, CaCl₂ precipitation, Hanahan method whose efficiency has been increased by using a reducing agent such as dimethyl sulfoxide (DMSO) in CaCl₂ precipitation, electroporation, calcium phosphate precipitation, protoplast fusion, agitation using silicon carbide fiber, Agrobacteria-mediated transformation, transformation using PEG, dextran sulfate-, Lipofectamine-, and dry/inhibition-mediated transformation, and the like may be used.

As described above, for optimization of properties of a fusion protein as a therapeutic agent or for any other purpose, glycosylation pattern of the fusion protein (for example, sialic acids, fucosylations, glycosylations) may be adjusted by manipulating, through methods known to those of ordinary skill in the art, glycosylation-related genes possessed by host cells.

### Method for producing a fusion protein

In another aspect of the present invention, there is provided a method for producing a fusion protein comprising an antibody fragment that specifically binds to C3b or C5; and a protein that specifically binds to VEGF, the method comprising the step of culturing the transformed cells. Specifically, the production method may comprise the steps of: i) culturing the transformed cells to obtain a culture product; and ii) recovering the fusion protein from the culture product.

The method of culturing the transformed cells may be carried out using methods well known in the art. Specifically, the culture may be carried out in a batch process, or carried out continuously in a fed batch or repeated fed batch process.

### Use of a fusion protein or a dimer thereof

In another aspect of the present invention, there is provided a pharmaceutical composition for treating or preventing eye diseases, comprising a fusion protein comprising an antibody fragment that specifically binds to C3b or C5; and a protein that specifically binds to VEGF or a fusion protein dimer in which the two fusion proteins are attached to each other as an active ingredient.

The fusion protein and the fusion protein dimer are as described above.

As used herein, the term "eye disease" may collectively refer to diseases that occur in the eye. The eye disease may refer to an eye disease triggered or worsened by complement activity or angiogenesis, or an eye disease that includes excessive angiogenesis as a major condition. The eye disease may be any one selected from the group consisting of age-related macular degeneration (AMD), geographic atrophy (GA), choroidal neovascularization (CNV), uveitis, diabetic and other ischemia-related retinopathy, diabetic macular edema, pathologic myopia, von Hippel-Lindau disease, ocular histoplasmosis, central retinal vein occlusion (CRVO), corneal neovascularization, and retinal neovascularization.

A preferred dosage of the pharmaceutical composition varies depending on the patient's condition and body weight, the severity of disease, the form of drug, the route and duration of administration and may be appropriately selected by those of ordinary skill in the art. In the pharmaceutical composition for treating or preventing eye diseases of the present invention, the active ingredient may be contained in any amount (effective amount) depending on application, dosage form, blending purpose, and the like, as long as the active ingredient may exhibit an activity of treating eye diseases or, in particular, a therapeutic effect on macular degeneration. A conventional effective amount thereof will be determined within a range of 0.001% by weight to 20.0% by weight, based on the total weight of the composition. As used herein, the term "effective amount" refers to an amount of an active ingredient capable of inducing an effect of improving or treating the condition of eye diseases, and in particular, inducing an effect of improving or treating the condition of macular degeneration. Such an effective amount may be experimentally determined within the scope of common knowledge of those of ordinary skill in the art.

As used herein, the term "treatment" may be used to mean both therapeutic and prophylactic treatment. At this time, prophylaxis may be used to mean that a pathological condition or disease of a subject is alleviated or mitigated. In one embodiment, the term "treatment" includes both application or any form of administration for treating a disease in a mammal, including a human. In addition, the term includes inhibiting or slowing down a disease or disease progression; and includes meanings of restoring or repairing impaired or lost function so that a disease is partially or completely alleviated; stimulating inefficient processes; or alleviating a serious disease.

Pharmacokinetic parameters such as bioavailability and underlying parameters such as clearance rate may also affect efficacy. Therefore, "enhanced efficacy" (for example, improvement in efficacy) may be due to enhanced pharmacokinetic parameters and enhanced efficacy, which may be measured by comparing parameters such as clearance rate and treatment or improvement of eye diseases in test animals or human subjects.

As used herein, the term "therapeutically effective amount" or "pharmaceutically effective amount" refers to an amount of a compound or composition effective to prevent or treat the disease in question, which is sufficient to treat the disease at a reasonable benefit/risk ratio applicable to medical treatment and does not cause adverse effects. A level of the effective amount may be determined depending on factors including the patient's health condition, type and severity of disease, activity of drug, the patient's sensitivity to drug, mode of administration, time of administration, route of administration and excretion rate, duration of treatment, combined or simultaneously used drugs, and other factors well known in the medical field. In one embodiment, the therapeutically effective amount means an amount of drug effective to treat eye diseases.

At this time, the pharmaceutical composition may further comprise a pharmaceutically acceptable carrier. The pharmaceutically acceptable carrier may be any carrier as long as the carrier is a non-toxic substance suitable for delivery to a patient. Distilled water, alcohol, fat, wax, and inert solid may be contained as the carrier. A pharmaceutically acceptable adjuvant (buffer, dispersant) may also be contained in the pharmaceutical composition.

Specifically, by including a pharmaceutically acceptable carrier in addition to an active ingredient, the pharmaceutical composition may be prepared into a parenteral formulation depending on its route of administration using conventional methods known in the art. At this time, the term "pharmaceutically acceptable" means that the carrier does not have more toxicity than the subject to be applied (prescribed) may adapt while not inhibiting activity of the active ingredient.

When the pharmaceutical composition is prepared into a parenteral formulation, it may be made into preparations in the form of injections, transdermal patches, nasal inhalants, and suppositories with suitable carriers according to methods known in the art. In a case of being formulated into injections, sterile water, ethanol, polyol such as glycerol or propylene glycol, or a mixture thereof may be used as a suitable carrier; and an isotonic solution, such as Ringer's solution, phosphate buffered saline (PBS) containing triethanol amine or sterile water for injection, or 5% dextrose, or the like may preferably be used. Formulation of pharmaceutical compositions is known in the art, and reference may specifically be made to Remington's Pharmaceutical Sciences (19th ed., 1995) and the like. This document is considered part of the present specification.

A preferred dosage of the pharmaceutical composition may range from 0.01 µg/kg to 10 g/kg, or 0.01 mg/kg to 1 g/kg, per day, depending on the patient's condition, body weight, sex, age, severity of the patient, and route of administration. The dosage may be administered once a day or may be divided into several times a day. Such a dosage should not be construed as limiting the scope of the present invention in any aspect.

Subjects to which the pharmaceutical composition may be applied (prescribed) are mammals and humans, with humans being particularly preferred. In addition to the active ingredient, the pharmaceutical composition of the present invention may further comprise any compound or natural extract, which is known to have a therapeutic effect on eye diseases, particularly macular degeneration.

In another aspect of the present invention, there is provided a use of a fusion protein comprising an antibody fragment that specifically binds to C3b or C5; and a protein that specifically binds to VEGF or a dimer thereof for manufacture of a medicament for treating or preventing eye diseases.

In another aspect of the present invention, there is provided a method for treating and/or preventing eye diseases, comprising administering a fusion protein comprising an antibody fragment that specifically binds to C3b or C5; and a protein that specifically binds to VEGF or a dimer thereof to a subject.

The fusion protein, the fusion protein dimer in which the two fusion proteins are attached to each other, and the eye disease are as described above. At this time, the subject may be a subject suffering from an eye disease. In addition, the subject may be a mammal, and preferably may be a human.

Route of administration, dosage, and frequency of administration of the fusion protein or the fusion protein dimer may vary depending on the patient's condition and the presence or absence of side effects, and thus the fusion protein or the fusion protein dimer may be administered to a subject in various ways and amounts. The optimal administration method, dosage, and frequency of administration may be selected in an appropriate range by those of ordinary skill in the art. In addition, the fusion protein or the fusion protein dimer may be administered in combination with other drugs or physiologically active substances whose therapeutic effect is known with respect to a disease to be treated, or may be formulated in the form of combination preparations with other drugs.

### Mode for Carrying out the Invention

Hereinafter, the present invention will be described in more detail by way of the following examples. However, the following examples are only for illustrating the present invention, and the scope of the present invention is not limited thereto.

### Preparation Example 1. Preparation of fusion protein

**[Table 1]**

| **PROTEIN** | **Format** | **Description** | **Corresponding sequence** |
|---|---|---|---|
| MOR09611 | Mab | anti-C3b (MOR09611) HC | SEQ ID NO: 1 |
| | | anti-C3b (MOR09611) LC | SEQ ID NO: 2 |
| MOR09675 | Mab | anti-C3b (MOR09675) HC | SEQ ID NO: 3 |
| | | anti-C3b (MOR09675) LC | SEQ ID NO: 4 |
| S77 | Mab | anti-C3b (S77) HC-hu IgG1 Fc DANG | SEQ ID NO: 5 |
| | | anti-C3b (S77) LC | SEQ ID NO: 6 |
| Eculizumab | Mab | anti-C5 (Eculizumab) HC-hu IgG1 Fc DANG | SEQ ID NO: 7 |
| | | anti-C5 (Eculizumab) LC | SEQ ID NO: 8 |
| PRO236 | Fc-fusion | anti-C3b (MOR09611) HC-hu IgG1 Fc DANG-VEGF binder | SEQ ID NO: 9 |
| | | anti-C3b (MOR09611) LC | SEQ ID NO: 2 |
| PRO237 | Fc-fusion | anti-C3b (MOR09675) HC-hu IgG1 Fc DANG-VEGF binder | SEQ ID NO: 10 |
| | | anti-C3b (MOR09675) LC | SEQ ID NO: 4 |
| PRO238 | Fc-fusion | anti-C3b (S77) HC-hu IgG1 Fc DANG-VEGF binder | SEQ ID NO: 11 |
| | | anti-C3b (S77) LC | SEQ ID NO: 6 |
| PRO239 | Fc-fusion | anti-C5 (Eculizumab) HC-hu IgG1 Fc DANG-VEGF binder | SEQ ID NO: 12 |
| | | anti-C5 (Eculizumab) LC | SEQ ID NO: 8 |
| PRO240 | Fc-fusion | VEGF binder-hu IgG1 Fc DANG-anti-C3b (MOR09611) scFv | SEQ ID NO: 13 |
| PRO241 | Fc-fusion | VEGF binder-hu IgG1 Fc DANG-anti-C3b (MOR09675) scFv | SEQ ID NO: 14 |
| PR0242 | Fc-fusion | VEGF binder-hu IgG1 Fc DANG-anti-C3b (S77) scFv | SEQ ID NO: 15 |
| PRO243 | Fc-fusion | VEGF binder-hu IgG1 Fc DANG-anti-C5 (Eculizumab) scFv | SEQ ID NO: 16 |
| PRO017 | Fc-fusion | hu IgG1 Fc DANG | SEQ ID NO: 17 |
| KNP-301 | Fc-fusion | hu CRIg-hu IgG1 Fc DANG-VEGF binder | SEQ ID NO: 18 |
| Aflibercept | Fc-fusion | VEGF binder-hu IgG1 Fc | SEQ ID NO: 19 |

[SEQ ID NO: 1] is the heavy chain sequence of the human anti-C3b antibody MOR09611.
[SEQ ID NO: 2] is the light chain sequence of the human anti-C3b antibody MOR09611.
[SEQ ID NO: 3] is the heavy chain sequence of the human anti-C3b antibody MOR09675.
[SEQ ID NO: 4] is the light chain sequence of the human anti-C3b antibody MOR09675.
[SEQ ID NO: 5] is composed of the heavy chain variable region sequence of the human anti-C3b antibody S77, the human IgG1 CH1 region sequence, and the human IgG1 Fc whose effector function has been removed through the DANG mutation (D265A, N297G).
[SEQ ID NO: 6] is the light chain sequence of the human anti-C3b antibody S77.
[SEQ ID NO: 7] is composed of the heavy chain variable region sequence of the human anti-C5 (anti-C5) antibody eculizumab, the human IgG1 CH1 region sequence, and the human IgG1 Fc DANG.
[SEQ ID NO: 8] is the light chain sequence of the human anti-C5 antibody eculizumab.
[SEQ ID NO: 9] is composed of the heavy chain variable region sequence of the human anti-C3b antibody MOR09611, the human IgG1 CH1 region sequence, the human IgG1 Fc DANG, the linker GGGGSGGGGS (SEQ ID NO: 59), and the VEGF binding site of aflibercept.
[SEQ ID NO: 10] is composed of the heavy chain variable region sequence of the human anti-C3b antibody MOR09675, the human IgG1 CH1 region sequence, the human IgG1 Fc DANG, the linker GGGGSGGGGS, and the VEGF binding site of aflibercept.
[SEQ ID NO: 11] is composed of the heavy chain variable region sequence of the human anti-C3b antibody S77, the human IgG1 CH1 region sequence, the human IgG1 Fc DANG, the linker GGGGSGGGGS, and the VEGF binding site of aflibercept.
[SEQ ID NO: 12] is composed of the heavy chain variable region sequence of the human anti-C5 antibody eculizumab, the human IgG1 CH1 region sequence, the human IgG1 Fc DANG, the linker GGGGSGGGGS, the the VEGF binding site of aflibercept.
[SEQ ID NO: 13] is composed of the VEGF binding site of aflibercept, the linker GGGGS (SEQ ID NO: 58), the human IgG1 Fc DANG, the linker GGGGSGGGGSGGGGS (SEQ ID NO: 60), the heavy chain variable region sequence of the human anti-C3b antibody MOR09611, the linker GGGGSGGGGSGGGGSGGGGS (SEQ ID NO: 61), and the light chain variable region sequence of MOR09611.
[SEQ ID NO: 14] is composed of the VEGF binding site of aflibercept, the linker GGGGS, the human IgG1 Fc DANG, the linker GGGGSGGGGSGGGGS, the heavy chain variable region sequence of the human anti-C3b antibody MOR09675, the linker GGGGSGGGGSGGGGSGGGGS, and the light chain variable region sequence of MOR09675.
[SEQ ID NO: 15] is composed of the VEGF binding site of aflibercept, the linker GGGGS, the human IgG1 Fc DANG, the linker GGGGSGGGGSGGGGS, the heavy chain variable region sequence of the human anti-C3b antibody S77, the linker GGGGSGGGGSGGGGSGGGGS, and the light chain variable region sequence of S77.
[SEQ ID NO: 16] is composed of the VEGF binding site of aflibercept, the linker GGGGS, the human IgG1 Fc DANG, the linker GGGGSGGGGSGGGGS, the light chain variable region sequence of the human anti-C5 antibody eculizumab, the linker GGGGSGGGGSGGGGS, and the heavy chain variable region sequence of eculizumab.
[SEQ ID NO: 17] is composed of the human IgG1 Fc DANG.
[SEQ ID NO: 18] is composed of the extracellular domain region (20-283) of the human CRIg protein, the linker GGGGS, and the human IgG1 Fc DANG.
[SEQ ID NO: 19] is composed of the sequence of aflibercept.
MOR09611 (SEQ ID NOs: 1 and 2) is a human anti-C3b antibody.
MOR09675 (SEQ ID NOs: 3 and 4) is a human anti-C3b antibody.
S77 (SEQ ID NOs: 5 and 6) is a human anti-C3b antibody.
Eculizumab (SEQ ID NOs: 7 and 8) is a human anti-C5 antibody.
PRO236 (SEQ ID NOs: 2 and 9) is a bispecific antibody with the effector function removed, in which the VEGF binding sites of the human anti-C3b antibody MOR09611 and aflibercept are located at the N-terminus and C-terminus, respectively.
PRO237 (SEQ ID NOs: 4 and 10) is a bispecific antibody with the effector function removed, in which the VEGF binding sites of the human anti-C3b antibody MOR09675 and aflibercept are located at the N-terminus and C-terminus, respectively.
PRO238 (SEQ ID NOs: 6 and 11) is a bispecific antibody with the effector function removed, in which the VEGF binding sites of the human anti-C3b antibody S77 and aflibercept are located at the N-terminus and C-terminus, respectively.
PRO239 (SEQ ID NOs: 8 and 12) is a bispecific antibody with the effector function removed, in which the VEGF binding sites of the human anti-C5 antibody eculizumab and aflibercept are located at the N-terminus and C-terminus, respectively.
PRO240 (SEQ ID NO: 13) is a bispecific antibody with the effector function removed, in which the VEGF binding site of aflibercept and the single chain variable region fragment (single chain fragment variable, scFv) sequence of the human anti-C3b antibody MOR09611 are located at the N-terminus and C-terminus, respectively.
PRO241 (SEQ ID NO: 14) is a bispecific antibody with the effector function removed, in which the VEGF binding site of aflibercept and the single chain variable region fragment sequence of the human anti-C3b antibody MOR09675 sequence are located at the N-terminus and C-terminus, respectively.
PRO242 (SEQ ID NO: 15) is a bispecific antibody with the effector function removed, in which the VEGF binding site of aflibercept and the single chain variable region fragment sequence of the human anti-C3b antibody S77 are located at the N-terminus and C-terminus, respectively.
PRO243 (SEQ ID NO: 16) is a bispecific antibody with the effector function removed, in which the VEGF binding site of aflibercept and the single chain variable region fragment sequence of the human anti-C5 antibody eculizumab are located at the N-terminus and C-terminus, respectively.
KNP-301 (SEQ ID NO: 18) is an Fc-fusion protein dimer with the effector function removed, in which the extracellular domain region of the human CRIg and the VEGF binding site of aflibercept are located at the N-terminus and C-terminus, respectively.
[SEQ ID NO: 1] anti-C3b (MOR09611) HC 
EVQLVESGGGLVQPGGSLRLSCAASGFTFSSYWMTWVRQAPGKGLEWVSSIKIKPDGYAASVKGRFTI

SRDNSKNTLYLQMNSLRAEDTAVYYCARLFYQYFARMDYWGQGTLVTVSSASTKGPSVFPLAPSSKSTSGGTAAL

GCLVKDYFPEPVTVSWNSGALTSGVHTFPAVLQSSGLYSLSSVVTVPSSSLGTQTYICNVNHKPSNTKVDKRVEP

KSCDKTHTCPPCPAPEAAGGPSVFLFPPKPKDTLMISRTPEVTCVVVDVSHEDPEVKFNWYVDGVEVHNAKTKPR

EEQYNSTYRVVSVLTVLHQDWLNGKEYKCKVSNKALPAPIEKTISKAKGQPREPQVYTLPPSREEMTKNQVSLTC

LVKGFYPSDIAVEWESNGQPENNYKTTPPVLDSDGSFFLYSKLTVDKSRWQQGNVFSCSVMHEALHNHYTQKSLS

LSPGK
[SEQ ID NO: 2] anti-C3b (MOR09611) LC 
DIQMTQSPSSLSASVGDRVTITCRASQDISNYLNWYQQKPGKAPKLLIYAASNLQSGVPSRFSGSGSG

TDFTLTISSLQPEDFATYYCQQHDTFRPTFGQGTKVEIKRTVAAPSVFIFPPSDEQLKSGTASVVCLLNNFYPRE

AKVQWKVDNALQSGNSQESVTEQDSKDSTYSLSSTLTLSKADYEKHKVYACEVTHQGLSSPVTKSFNRGEC
[SEQ ID NO: 3] anti-C3b (MOR09675) HC 
EVQLVQSGAEVKKPGASVKVSCKASGYTFTGYYINWVRQAPGQGLEWMGIISPNQGTTGYAQKFQGRV

TMTRDTSISTAYMELSSLRSEDTAVYYCARGNYDHLDYWGQGTLVTVSSASTKGPSVFPLAPSSKSTSGGTAALG

CLVKDYFPEPVTVSWNSGALTSGVHTFPAVIQSSGLYSLSSVVTVPSSSLGTQTYICNVNHKPSNTKVDKRVEPK

SCDKTHTCPPCPAPEAAGGPSVFLFPPKPKDTLMISRTPEVTCVVVDVSHEDPEVKFNWYVDGVEVHNAKTKPRE

EQYNSTYRVVSVLTVLHQDWLNGKEYKCKVSNKALPAPIEKTISKAKGQPREPQVYTLPPSREEMTKNQVSLTCL

VKGFYPSDIAVEWESNGQPENNYKTTPPVLDSDGSFFLYSKLTVDKSRWQQGNVFSCSVMHEALHNHYTQKSLSL

SPGK
[SEQ ID NO: 4] anti-C3b (MOR09675) LC 
DIQMTQSPSSLSASVGDRVTITCRASQGISNYLNWYQQKPGKAPKLLIYDASTLQSGVPSRFSGSGSG

TDFTLTISSLQPEDFATYYCQQDWHTLPVTFGQGTKVEIKRTVAAPSVFIFPPSDEQLKSGTASVVCLLNNFYPR

EAKVQWKVDNALQSGNSQESVTEQDSKDSTYSLSSTLTLSKADYEKHKVYACEVTHQGLSSPVTKSFNRGEC
[SEQ ID NO: 5] anti-C3b (S77) HC-hu IgG1 Fc DANG 
EVQLVESGGGLVQPGGSLRLSCAASGFSFTSSSVSWVRQAPGKGLEWVGLIYPYNGFNYYADSVKGRF

TISADTSKNTAYLQMNSLRAEDTAVYYCARNALYGSGGYYAMDYWGQGTLVTVSSASTKGPSVFPLAPSSKSTSG

GTAALGCLVKDYFPEPVTVSWNSGALTSGVHTFPAVIQSSGLYSLSSVVTVPSSSLGTQTYICNVNHKPSNTKVD

KRVEPKSCDKTHTCPPCPAPEAAGGPSVFLFPPKPKDTLMISRTPEVTCVVVAVSHEDPEVKFNWYVDGVEVHNA

KTKPREEQYGSTYRVVSVLTVLHQDWLNGKEYKCKVSNKALPAPIEKTISKAKGQPREPQVYTLPPSREEMTKNQ

VSLTCLVKGFYPSDIAVEWESNGQPENNYKTTPPVLDSDGSFFLYSKLTVDKSRWQQGNVFSCSVMHEALHNHYT

QKSLSLSPGK
[SEQ ID NO: 6] anti-C3b (S77) LC 
DIQMTQSPSSLSASVGDRVTITCRASQDVSTAVAWYQQKPGKAPKLLIYSASFLYSGVPSRFSGSGSG

TDFTLTISSLQPEDFATYYCQQSYATLPTFEQGTKVEIKRTVAAPSVFIFPPSDEQLKSGTASVVCLLNNFYPRE

AKVQWKVDNALQSGNSQESVTEQDSKDSTYSLSSTLTLSKADYEKHKVYACEVTHQGLSSPVTKSFNRGEC
[SEQ ID NO: 7] anti-C5 (Eculizumab) HC-hu IgG1 Fc DANG 
QVQLVQSGAEVKKPGASVKVSCKASGYIFSNYWIQWVRQAPGQGLEWMGEILPGSGSTEYTENFKDRV
TMTRDTSTSTVYMELSSLRSEDTAVYYCARYFFGSSPNWYFDVWGQGTLVTVSSASTKGPSVFPLAPSSKSTSGG
TAALGCLVKDYFPEPVTVSWNSGALTSGVHTFPAVIQSSGLYSLSSVVTVPSSSLGTQTYICNVNHKPSNTKVDK
RVEPKSCDKTHTCPPCPAPEAAGGPSVFLFPPKPKDTLMISRTPEVTCVVVAVSHEDPEVKFNWYVDGVEVHNAK
TKPREEQYGSTYRVVSVLTVLHQDWLNGKEYKCKVSNKALPAPIEKTISKAKGQPREPQVYTLPPSREEMTKNQV
SLTCLVKGFYPSDIAVEWESNGQPENNYKTTPPVLDSDGSFFLYSKLTVDKSRWQQGNVFSCSVMHEALHNHYTQ
KSLSLSPGK
[SEQ ID NO: 8] anti-C5 (Eculizumab) LC
[SEQ ID NO: 9] anti-C3b (MOR09611) HC-hu IgG1 Fc DANG-VEGF binder 
EVQLVESGGGLVQPGGSLRLSCAASGFTFSSYWMTWVRQAPGKGLEWVSSIKIKPDGYAASVKGRFTI

SRDNSKNTLYLQMNSLRAEDTAVYYCARLFYQYFARMDYWGQGTLVTVSSASTKGPSVFPLAPSSKSTSGGTAAL

GCLVKDYFPEPVTVSWNSGALTSGVHTFPAVLQSSGLYSLSSVVTVPSSSLGTQTYICNVNHKPSNTKVDKRVEP

KSCDKTHTCPPCPAPEAAGGPSVFLFPPKPKDTLMISRTPEVTCVVVAVSHEDPEVKFNWYVDGVEVHNAKTKPR

EEQYGSTYRVVSVLTVLHQDWLNGKEYKCKVSNKALPAPIEKTISKAKGQPREPQVYTLPPSREEMTKNQVSLTC

LVKGFYPSDIAVEWESNGQPENNYKTTPPVLDSDGSFFLYSKLTVDKSRWQQGNVFSCSVMHEALHNHYTQKSLS

LSPGGGGGSGGGGSSDTGRPFVEMYSEIPEIIHMTEGRELVIPCRVTSPNITVTLKKFPLDTLIPDGKRIIWDSR

KGFIISNATYKEIGLLTCEATVNGHLYKTNYLTHRQTNTIIDVVLSPSHGIELSVGEKLVLNCTARTELNVGIDF

NWEYPSSKHQHKKLVNRDLKTQSGSEMKKFLSTLTIDGVTRSDQGLYTCAASSGLMTKKNSTFVRVHEK
[SEQ ID NO: 10] anti-C3b (MOR09675) HC-hu IgG1 Fc DANG-VEGF binder 
EVQLVQSGAEVKKPGASVKVSCKASGYTFTGYYINWVRQAPGQGLEWMGIISPNQGTTGYAQKFQGRV

TMTRDTSISTAYMELSSLRSEDTAVYYCARGNYDHLDYWGQGTLVTVSSASTKGPSVFPLAPSSKSTSGGTAALG

CLVKDYFPEPVTVSWNSGALTSGVHTFPAVIQSSGLYSLSSVVTVPSSSLGTQTYICNVNHKPSNTKVDKRVEPK

SCDKTHTCPPCPAPEAAGGPSVFLFPPKPKDTLMISRTPEVTCVVVAVSHEDPEVKFNWYVDGVEVHNAKTKPRE

EQYGSTYRVVSVLTVLHQDWLNGKEYKCKVSNKALPAPIEKTISKAKGQPREPQVYTLPPSREEMTKNQVSLTCL

VKGFYPSDIAVEWESNGQPENNYKTTPPVLDSDGSFFLYSKLTVDKSRWQQGNVFSCSVMHEALHNHYTQKSLSL

SPGGGGGSGGGGSSDTGRPFVEMYSEIPEIIHMTEGRELVIPCRVTSPNITVTLKKFPLDTLIPDGKRIIWDSRK

GFIISNATYKEIGLLTCEATVNGHLYKTNYLTHRQTNTIIDVVLSPSHGIELSVGEKLVLNCTARTELNVGIDFN

WEYPSSKHQHKKLVNRDLKTQSGSEMKKFLSTLTIDGVTRSDQGLYTCAASSGLMTKKNSTFVRVHEK
[SEQ ID NO: 11] anti-C3b (S77) HC-hu IgG1 Fc DANG-VEGF binder 
EVQLVESGGGLVQPGGSLRLSCAASGFSFTSSSVSWVRQAPGKGLEWVGLIYPYNGFNYYADSVKGRF

TISADTSKNTAYLQMNSLRAEDTAVYYCARNALYGSGGYYAMDYWGQGTLVTVSSASTKGPSVFPLAPSSKSTSG

GTAALGCLVKDYFPEPVTVSWNSGALTSGVHTFPAVIQSSGLYSLSSVVTVPSSSLGTQTYICNVNHKPSNTKVD

KRVEPKSCDKTHTCPPCPAPEAAGGPSVFLFPPKPKDTLMISRTPEVTCVVVAVSHEDPEVKFNWYVDGVEVHNA

KTKPREEQYGSTYRVVSVLTVLHQDWLNGKEYKCKVSNKALPAPIEKTISKAKGQPREPQVYTLPPSREEMTKNQ

VSLTCLVKGFYPSDIAVEWESNGQPENNYKTTPPVLDSDGSFFLYSKLTVDKSRWQQGNVFSCSVMHEALHNHYT

QKSLSLSPGGGGGSGGGGSSDTGRPFVEMYSEIPEIIHMTEGRELVIPCRVTSPNITVTLKKFPLDTLIPDGKRI

IWDSRKGFIISNATYKEIGLLTCEATVNGHLYKTNYLTHRQTNTIIDVVLSPSHGIELSVGEKLVLNCTARTELN

VGIDFNWEYPSSKHQHKKLVNRDLKTQSGSEMKKFLSTLTIDGVTRSDQGLYTCAASSGLMTKKNSTFVRVHEK
[SEQ ID NO: 12] anti-C5 (Eculizumab) HC-hu IgG1 Fc DANG-VEGF binder 
QVQLVQSGAEVKKPGASVKVSCKASGYIFSNYWIQWVRQAPGQGLEWMGEILPGSGSTEYTENFKDRV

TMTRDTSTSTVYMELSSLRSEDTAVYYCARYFFGSSPNWYFDVWGQGTLVTVSSASTKGPSVFPLAPSSKSTSGG

TAALGCLVKDYFPEPVTVSWNSGALTSGVHTFPAVIQSSGLYSLSSVVTVPSSSLGTQTYICNVNHKPSNTKVDK

RVEPKSCDKTHTCPPCPAPEAAGGPSVFLFPPKPKDTLMISRTPEVTCVVVAVSHEDPEVKFNWYVDGVEVHNAK

TKPREEQYGSTYRVVSVLTVLHQDWLNGKEYKCKVSNKALPAPIEKTISKAKGQPREPQVYTLPPSREEMTKNQV

SLTCLVKGFYPSDIAVEWESNGQPENNYKTTPPVLDSDGSFFLYSKLTVDKSRWQQGNVFSCSVMHEALHNHYTQ

KSLSLSPGGGGGSGGGGSSDTGRPFVEMYSEIPEIIHMTEGRELVIPCRVTSPNITVTLKKFPLDTLIPDGKRII

WDSRKGFIISNATYKEIGLLTCEATVNGHLYKTNYLTHRQTNTIIDVVLSPSHGIELSVGEKLVLNCTARTELNV

GIDFNWEYPSSKHQHKKLVNRDLKTQSGSEMKKFLSTLTIDGVTRSDQGLYTCAASSGLMTKKNSTFVRVHEK
[SEQ ID NO: 13] VEGF binder-hu IgG1 Fc DANG-anti-C3b (MOR09611) scFv 
SDTGRPFVEMYSEIPEIIHMTEGRELVIPCRVTSPNITVTLKKFPLDTLIPDGKRIIWDSRKGFIISN
ATYKEIGLLTCEATVNGHLYKTNYLTHRQTNTIIDVVLSPSHGIELSVGEKLVLNCTARTELNVGIDFNWEYPSS
KHQHKKLVNRDLKTQSGSEMKKFLSTLTIDGVTRSDQGLYTCAASSGLMTKKNSTFVRVHEKGGGGSDKTHTCPP
CPAPEAAGGPSVFLFPPKPKDTLMISRTPEVTCVVVAVSHEDPEVKFNWYVDGVEVHNAKTKPREEQYGSTYRVV
SVLTVLHQDWLNGKEYKCKVSNKALPAPIEKTISKAKGQPREPQVYTLPPSREEMTKNQVSLTCLVKGFYPSDIA
VEWESNGQPENNYKTTPPVLDSDGSFFLYSKLTVDKSRWQQGNVFSCSVMHEALHNHYTQKSLSLSPGGGGGSGG
GGSGGGGSEVQLVESGGGLVQPGGSLRLSCAASGFTFSSYWMTWVRQAPGKGLEWVSSIKIKPDGYAASVKGRFT
ISRDNSKNTLYLQMNSLRAEDTAVYYCARLFYQYFARMDYWGQGTLVTVSSGGGGSGGGGSGGGGSGGGGSDIQM
TQSPSSLSASVGDRVTITCRASQDISNYLNWYQQKPGKAPKLLIYAASNLQSGVPSRFSGSGSGTDFTLTISSLQ
PEDFATYYCQQHDTFRPTFGQGTKVEIK
[SEQ ID NO: 14] VEGF binder-hu IgG1 Fc DANG-anti-C3b (MOR09675) scFv 
SDTGRPFVEMYSEIPEIIHMTEGRELVIPCRVTSPNITVTLKKFPLDTLIPDGKRIIWDSRKGFIISN

ATYKEIGLLTCEATVNGHLYKTNYLTHRQTNTIIDVVLSPSHGIELSVGEKLVLNCTARTELNVGIDFNWEYPSS

KHQHKKLVNRDLKTQSGSEMKKFLSTLTIDGVTRSDQGLYTCAASSGLMTKKNSTFVRVHEKGGGGSDKTHTCPP

CPAPEAAGGPSVFLFPPKPKDTLMISRTPEVTCVVVAVSHEDPEVKFNWYVDGVEVHNAKTKPREEQYGSTYRVV

SVLTVLHQDWLNGKEYKCKVSNKALPAPIEKTISKAKGQPREPQVYTLPPSREEMTKNQVSLTCLVKGFYPSDIA

VEWESNGQPENNYKTTPPVLDSDGSFFLYSKLTVDKSRWQQGNVFSCSVMHEALHNHYTQKSLSLSPGGGGGSGG

GGSGGGGSEVQLVQSGAEVKKPGASVKVSCKASGYTFTGYYINWVRQAPGQGLEWMGIISPNQGTTGYAQKFQGR

VTMTRDTSISTAYMELSSLRSEDTAVYYCARGNYDHLDYWGQGTLVTVSSGGGGSGGGGSGGGGSGGGGSDIQMT

QSPSSLSASVGDRVTITCRASQGISNYLNWYQQKPGKAPKLLIYDASTLQSGVPSRFSGSGSGTDFTLTISSLQP

EDFATYYCQQDWHTLPVTFGQGTKVEIK
[SEQ ID NO: 15] VEGF binder-hu IgG1 Fc DANG-anti-C3b (S77) scFv 
SDTGRPFVEMYSEIPEIIHMTEGRELVIPCRVTSPNITVTLKKFPLDTLIPDGKRIIWDSRKGFIISN

ATYKEIGLLTCEATVNGHLYKTNYLTHRQTNTIIDVVLSPSHGIELSVGEKLVLNCTARTELNVGIDFNWEYPSS

KHQHKKLVNRDLKTQSGSEMKKFLSTLTIDGVTRSDQGLYTCAASSGLMTKKNSTFVRVHEKGGGGSDKTHTCPP

CPAPEAAGGPSVFLFPPKPKDTLMISRTPEVTCVVVAVSHEDPEVKFNWYVDGVEVHNAKTKPREEQYGSTYRVV

SVLTVLHQDWLNGKEYKCKVSNKALPAPIEKTISKAKGQPREPQVYTLPPSREEMTKNQVSLTCLVKGFYPSDIA

VEWESNGQPENNYKTTPPVLDSDGSFFLYSKLTVDKSRWQQGNVFSCSVMHEALHNHYTQKSLSLSPGGGGGSGG

GGSGGGGSEVQLVESGGGLVQPGGSLRLSCAASGFSFTSSSVSWVRQAPGKGLEWVGLIYPYNGFNYYADSVKGR

FTISADTSKNTAYLQMNSLRAEDTAVYYCARNALYGSGGYYAMDYWGQGTLVTVSSGGGGSGGGGSGGGGSGGGG

SDIQMTQSPSSLSASVGDRVTITCRASQDVSTAVAWYQQKPGKAPKLLIYSASFLYSGVPSRFSGSGSGTDFTLT

ISSLQPEDFATYYCQQSYATLPTFGQGTKVEIK
[SEQ ID NO: 16] VEGF binder-hu IgG1 Fc DANG-anti-C5 (Eculizumab) scFv 
SDTGRPFVEMYSEIPEIIHMTEGRELVIPCRVTSPNITVTLKKFPLDTLIPDGKRIIWDSRKGFIISN

ATYKEIGLLTCEATVNGHLYKTNYLTHRQTNTIIDVVLSPSHGIELSVGEKLVLNCTARTELNVGIDFNWEYPSS

KHQHKKLVNRDLKTQSGSEMKKFLSTLTIDGVTRSDQGLYTCAASSGLMTKKNSTFVRVHEKGGGGSDKTHTCPP

CPAPEAAGGPSVFLFPPKPKDTLMISRTPEVTCVVVAVSHEDPEVKFNWYVDGVEVHNAKTKPREEQYGSTYRVV

SVLTVLHQDWLNGKEYKCKVSNKALPAPIEKTISKAKGQPREPQVYTLPPSREEMTKNQVSLTCLVKGFYPSDIA

VEWESNGQPENNYKTTPPVLDSDGSFFLYSKLTVDKSRWQQGNVFSCSVMHEALHNHYTQKSLSLSPGGGGGSGG

GGSGGGGSMADIQMTQSPSSLSASVGDRVTITCGASENIYGALNWYQQKPGKAPKLLIYGATNLADGVPSRFSGS

GSGTDFTLTISSLQPEDFATYYCQNVLNTPLTFGQGTKVEIKRTGGGGSGGGGSGGGGSQVQLVQSGAEVKKPGA

SVKVSCKASGYIFSNYWIQWVRQAPGQGLEWMGEILPGSGSTEYTENFKDRVTMTRDTSTSTVYMELSSLRSEDT

AVYYCARYFFGSSPNWYFDVWGQGTLVTVSS
[SEQ ID NO: 17] hu IgG1 Fc DANG 
DKTHTCPPCPAPELLGGPSVFLFPPKPKDTLMISRTPEVTCVVVAVSHEDPEVKFNWYVDGVEVHNAK
TKPREEQYGSTYRVVSVLTVLHQDWLNGKEYKCKVSNKALPAPIEKTISKAKGQPREPQVYTLPPSRDELTKNQV
SLTCLVKGFYPSDIAVEWESNGQPENNYKTTPPVLDSDGSFFLYSKLTVDKSRWQQGNVFSCSVMHEALHNHYTQ
KSLSLSPGK
[SEQ ID NO: 18] hu CRIg-hu IgG1 Fc DANG-VEGF binder 
RPILEVPESVTGPWKGDVNLPCTYDPLQGYTQVLVKWLVQRGSDPVTIFLRDSSGDHIQQAKYQGRLH

VSHKVPGDVSLQLSTLEMDDRSHYTCEVTWQTPDGNQVVRDKITELRVQKLSVSKPTVTTGSGYGFTVPQGMRIS

LQCQARGSPPISYIWYKQQTNNQEPIKVATLSTLLFKPAVIADSGSYFCTAKGQVGSEQHSDIVKFVVKDSSKLL

KTKTEAPTTMTYPLKATSTVKQSWDWTTDMDGYLGETSAGPGKSLPGGGGSDKTHTCPPCPAPELLGGPSVFLFP

PKPKDTLMISRTPEVTCVVVAVSHEDPEVKFNWYVDGVEVHNAKTKPREEQYGSTYRVVSVLTVLHQDWLNGKEY

KCKVSNKALPAPIEKTISKAKGQPREPQVYTLPPSRDELTKNQVSLTCLVKGFYPSDIAVEWESNGQPENNYKTT

PPVLDSDGSFFLYSKLTVDKSRWQQGNVFSCSVMHEALHNHYTQKSLSLSPGGGGGSGGGGSSDTGRPFVEMYSE

IPEIIHMTEGRELVIPCRVTSPNITVTLKKFPLDTLIPDGKRIIWDSRKGFIISNATYKEIGLLTCEATVNGHLY

KTNYLTHRQTNTIIDVVLSPSHGIELSVGEKLVLNCTARTELNVGIDFNWEYPSSKHQHKKLVNRDLKTQSGSEM

KKFLSTLTIDGVTRSDQGLYTCAASSGLMTKKNSTFVRVHEK
[SEQ ID NO: 19] VEGF binder-hu IgG1 Fc

SDTGRPFVEMYSEIPEIIHMTEGRELVIPCRVTSPNITVTLKKFPLDTLIPDGKRIIWDSRKGFIISN

ATYKEIGLLTCEATVNGHLYKTNYLTHRQTNTIIDVVLSPSHGIELSVGEKLVLNCTARTELNVGIDFNWEYPSS

KHQHKKLVNRDLKTQSGSEMKKFLSTLTIDGVTRSDQGLYTCAASSGLMTKKNSTFVRVHEKDKTHTCPPCPAPE

LLGGPSVFLFPPKPKDTLMISRTPEVTCVVVDVSHEDPEVKFNWYVDGVEVHNAKTKPREEQYNSTYRVVSVLTV

LHQDWLNGKEYKCKVSNKALPAPIEKTISKAKGQPREPQVYTLPPSRDELTKNQVSLTCLVKGFYPSDIAVEWES

NGQPENNYKTTPPVLDSDGSFFLYSKLTVDKSRWQQGNVFSCSVMHEALHNHYTQKSLSLSPGK

### Preparation Example 2. Preparation of proteins

### Preparation Example 2.1. Vector composition and plasmid maxi-prep

Reagents and equipment used are described in Tables 2 and 3 below.

**[Table 2]**

| **Reagent** | **Manufacturer** | **Catalog#** |
|---|---|---|
| pTT5 | chempartner | |
| In-Fusion HD cloning kit | Clontech | 639648 |
| Accuprime pfx DNA polymerase | Invitrogen | 12344-04 |
| Gel DNA fragment purification Kit | TaKaRa | D823A |
| FastDigest^{®} BamHI | Fermentas | FD0055 |
| FastDigest^{®} EcoRI | Fermentas | FD0275 |

**[Table 3]**

| **Equipment and tool** | **Manufacturer** | **Model name** |
|---|---|---|
| Biosafety cabinet | NUAIRE | LabGard class II |
| Centrifuge | Eppendorf | 5424 |
| Gel imaging system | Tanon | 2500R |

The synthesized DNA fragment was amplified through PCR, and the PCR product was purified by gel. The pTT5 vector was cleaved by the restriction enzymes EcoRI and BamHI, and then the gel was purified. Each PCR product and the linear vector were ligated using the In-Fusion kit. The produced vector was transformed into ECOS101 DH5α competent cells, and the cells were cultured on 2xYT agar plates containing 100 µg/ml ampicillin. All manipulation processes were performed according to standard transformation protocols. Positive recombinant products were identified by colony PCR, and sequence-verification sequencing was performed on the recombinant plasmid. A single colony was selected, and the seed culture was inoculated into 5 mL of 2xYT medium containing 100 µg/mL ampicillin. It was cultured with shaking at 37 °C for 8 hours.

Thereafter, the seed culture was diluted in 200 mL of selective 2xYT medium at a ratio of 1:1,000. It was cultured with shaking at 37 °C for 16 hours. Bacterial cells were harvested by centrifugation at 4 °C at 4,700 rpm for 10 minutes. The bacterial pellet was resuspended in 12 mL of RES-EF buffer. Thereafter, 12 mL of LYS-EF buffer was added, and the sealed tube was inverted vigorously to mix thoroughly and then incubated for 5 minutes at room temperature. 12 mL of NEU-EF buffer was added to the lysate, and inverted vigorously to mix thoroughly and rapidly. Before injecting the lysate into the NucleoBond^{®} Xtra column filter, a homogeneous suspension of the precipitate was prepared by inverting the lysate tube 3 times to prevent clogging of the filter.

Thereafter, the NucleoBond^{®} Xtra column filter and the NucleoBond^{®} Xtra column were washed with 10 mL of filter wash buffer FIL-EF. The NucleoBond^{®} Xtra column filter was removed by pulling out or inverting the column. The NucleoBond^{®} Xtra column was washed with 90 mL of wash buffer ENDO.

The NucleoBond^{®} Xtra column was washed with 45 mL of wash buffer WASH-EF. Plasmid DNA was eluted with 15 mL of elution buffer ELU. The eluate was collected in a 50 mL centrifugation tube. 10.5 mL of isopropanol at room temperature was added to precipitate the eluted plasmid DNA. After vortexing, the mixture was allowed to stand for 2 minutes.

Thereafter, 5 mL of 70% ethanol was added to the pellet. Ethanol was carefully and completely removed from the tube using a pipette tip. The pellet was dried at room temperature (20-25 °C). Thereafter, the DNA pellet was dissolved with 1,000 µL of purified water.

### Preparation Example 2.2. Cell transfection and protein expression

Materials and reagents used are described in Table 4 below.

**[Table 4]**

| **Material and reagent** | **Manufacturer (Product #)** |
|---|---|
| 293F cells | Invitrogen (R790-07) |
| OPM 293 | OPM (81075-001) |
| Pluronic^{®} F-68, 10% (100X) | Gibco (24040-032) |
| 1 mg/mL PEI | Polyscience (23966) |
| OPTI MEM I | Gibco (31985088) |
| Peptone (20x) | FLUKA (P0521-1KG) |
| Shaker flask | |
| ISF1-X incubator shaker | Kuhner shaker |

The 293F seed strain containing the complete medium was maintained in an incubator shaker at 130 rpm, 37 °C, and 8% CO₂. The cells were cultured at a density of 0.3 10⁶ to 0.4 × 10⁶ cells/mL, and the medium was changed every 2 to 3 days. Twenty-four hours before transfection, the freshly subcultured 293F cells were prepared at 2.6 × 10⁶ cells/mL. The prepared cells were cultured in an incubator shaker at 130 rpm, 37 °C, and 8% CO₂.

On the day of transfection, a density of cells was adjusted to a density of 5.0 × 10⁶ cells/mL using a fresh medium. It was performed at a total volume of 1 L in a 3 L shaker flask. 0.4 mg of HC and 0.6 mg of LC plasmids were diluted with 50 mL of OPTI MEM I and filtered through a 0.22 µm filter. Thereafter, 2 mg of PEI was diluted with 50 mL of OPTI MEM I to prepare a transfection reagent.

The diluted PEI was added to the DNA mixture and then mixed immediately. Thereafter, it was cultured for 15 minutes at room temperature. The DNA-PEI mixture was added to 293F cells prepared at 2.6 × 10⁶ cells/mL. Thereafter, the cells were continuously cultured for 24 hours in an incubator shaker at 130 rpm, 37°C, and 8% CO₂. Twenty-four hours after transfection, 10% peptone was added to 1/20 of the culture solution so that the final concentration was 0.5%. Thereafter, the cells were continuously cultured in an incubator shaker at 130 rpm, 37°C, and 8% CO₂. The cell density/viability was measured and recorded daily during the 2 to 5 days period after transfection. The cells were harvested for purification 7 days after transfection or when the cell viability was less than 70%.

### Preparation Example 2.3. Protein purification

Reagents, composition of each buffer, and equipment used for protein purification are described in Tables 5 to 7 below.

**[Table 5]**

| **Reagent** | **Manufacturer** | **Catalog#** |
|---|---|---|
| Mabselect SuRe | GE Healthcare | 11003493 |
| Tris | SIGMA | 77-86-1 |
| NaCl | ACROS ORGANIVS | 7647-14-5 |
| Sodium citrate | Adamas-beta | 76198B |
| Citric acid | GENERAL-Reagent | G83162B |
| Arginine | VETEC | V900343-500G |
| Succinic acid | Sigma-Aldrich | S9512-500G |
| Triton X-100 | ABCONE | X10010-1L |
| Triton X-114 | SIGMA-ALDRICH | X114 |
| Millex-GP Filter Unit 0.22 µm Sterile | MILLIPORE | SLGP033RS |
| NaOH | Merck | B146369740 |

**[Table 6]**

| | |
|---|---|
| Buffer A | 25 mM Tris, 150 mM NaCl, pH 8.0 |
| Buffer B | 25 mM Tris, 150 mM NaCl,0.1% Triton X-100, 0.1% Triton X-114 pH 8.0 |
| Buffer C | 100 mM Sodium Citrate, 150 mM NaCl, pH 3.0 |
| Buffer D | 1 M Arginine, 400 mM Succinic acid, pH 9.0 |
| Final buffer | 10 mM PB, pH 7.4, 40 mM NaCl, 5% sucrose |

**[Table 7]**

| **Equipment** | **Manufacturer** | **Model name** |
|---|---|---|
| AKTA Pure | GE Healthcare | 29-0182-24 |
| Centrifuge | Beckman | J-26xp |
| Gel imaging system | Tanon | 2500R |
| Sartopore 2 filter | Sartorius | 5445307H9-OO-A |

The proteins were purified using a MabSelect Sure column. Specifically, the supernatant was harvested by centrifugation at 2,000×g at 4 °C for 20 minutes. Thereafter, the supernatant was filtered with a Sartopore 2 filter. A 5 ml of MabSelect Sure column equilibrated with Buffer A was loaded with the clarified supernatant. Thereafter, the column was washed with Buffer A until the A280 absorbance reached the baseline. In addition, the column was washed with 10 CV Buffer B. In addition, the column was washed with 10 CV Buffer A. Thereafter, the bound proteins were eluted with 6 CV Buffer C, and 1/6 volume of Buffer D was added to neutralize the eluted substance. Thereafter, SDS-PAGE and SEC-HPLC analysis were performed.

Thereafter, the proteins were purified using a SEC column. Specifically, the supernatant was loaded onto the SEC column equilibrated with the final buffer, and then the proteins were eluted with the final buffer.

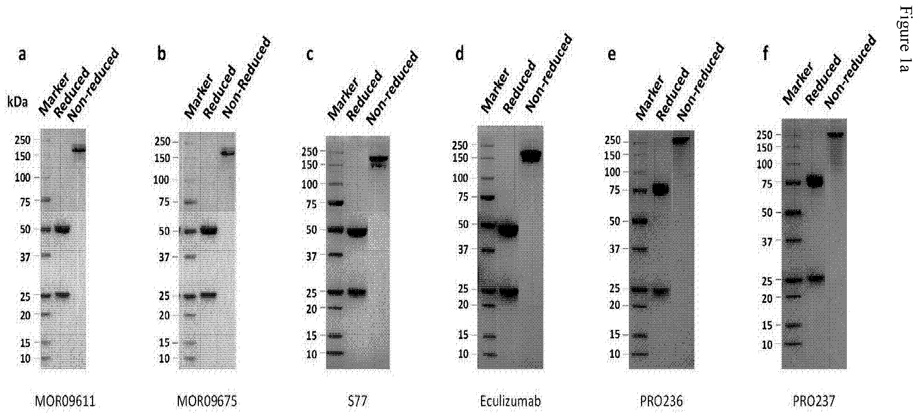

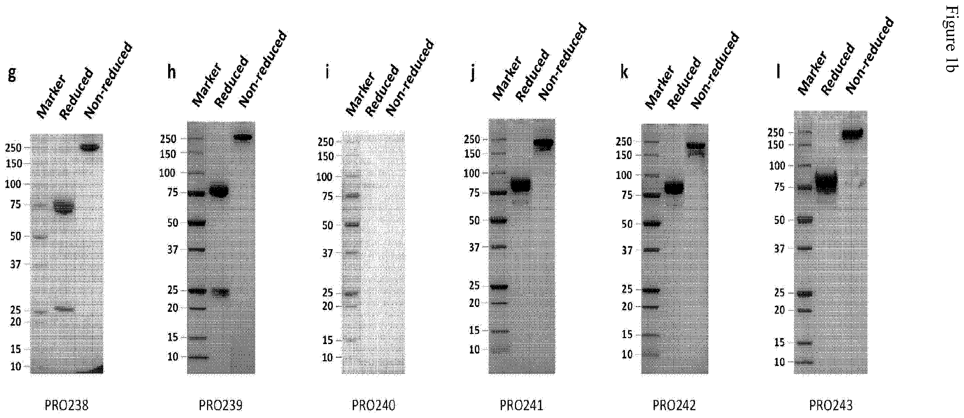

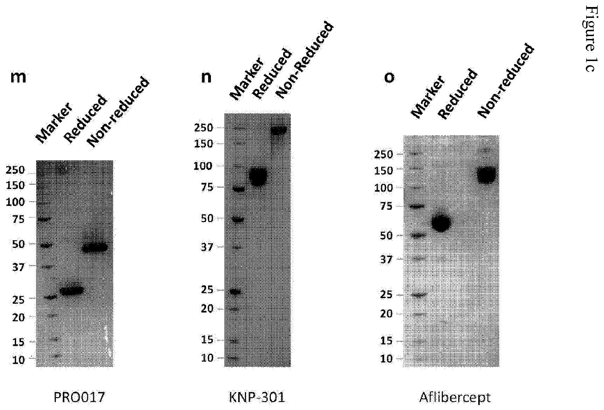

As a result, as shown in Figures 1a to 1c, the purified fusion protein dimers MOR09611, MOR09675, S77, eculizumab, PRO236, PRO237, PRO238, PRO239, PRO240, PRO241, PRO242, PRO243, PRO017, KNP-301, and aflibercept were identified by SDS-PAGE.

### Experimental Example 1. Identification of binding affinity of fusion protein to complement protein and VEGF through ELISA

In order to determine whether the fusion protein dimer of one embodiment inhibits the complement pathway, through an enzyme immunoassay, it was determined whether KNP-301, MOR09611, MOR09675, PRO236, PRO237, PRO238, PRO239, PRO241, PRO242, S77, and eculizumab bind to the C3b protein, and whether PRO239, eculizumab, and PRO017 bind to C5.

Specifically, the human C3b protein was immobilized on a plate, and KNP-301, MOR09611, MOR09675, PRO236, PRO237, PRO238, PRO239, PRO241, PRO242, S77, and eculizumab were allowed to bind. Next, the anti-human immunoglobulin G antibody and the anti-horseradish peroxidase (HRP) antibody were sequentially allowed to bind. In addition, the human C5 protein was immobilized on a plate, and PRO239, eculizumab, and PRO017 were allowed to bind to human C5.

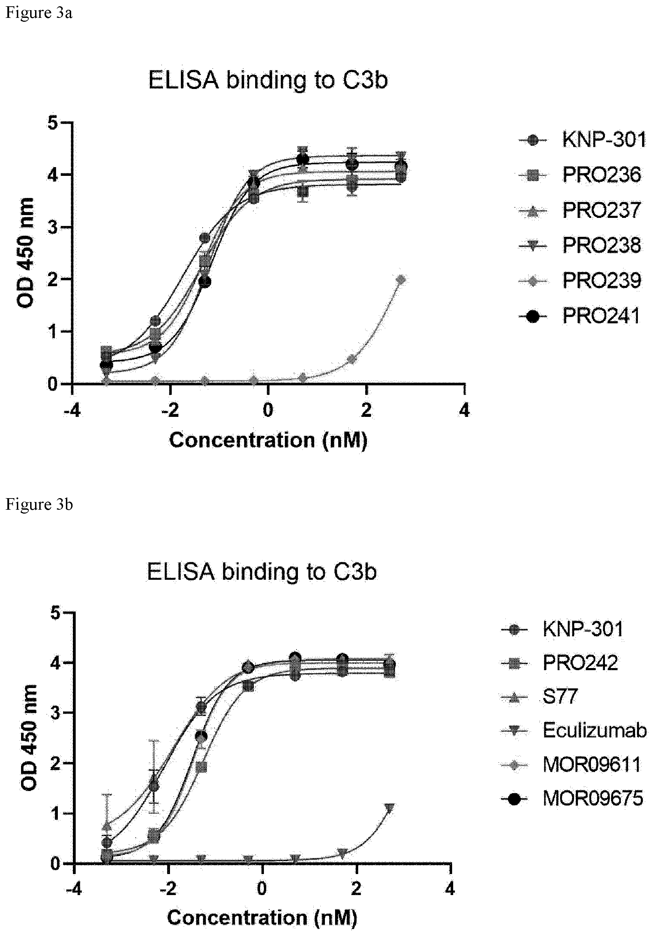

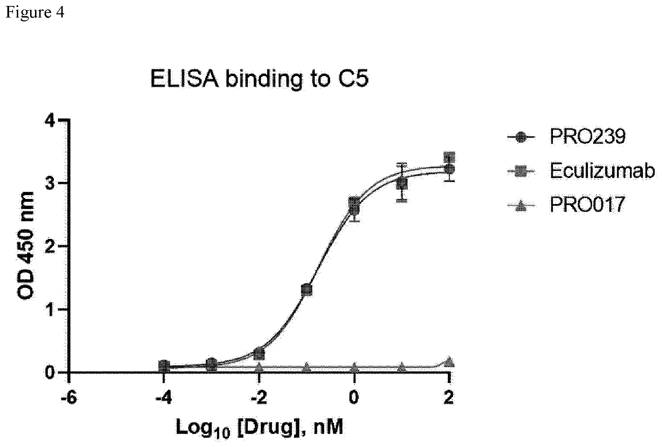

As a result, as shown in Figures 3a and 3b, it was found that KNP-301, MOR09611, MOR09675, PRO236, PRO237, PRO238, PRO241, PRO242, and S77 bind to human C3b in a concentration-dependent manner. In addition, as shown in Figure 4, it was found that PRO239 and eculizumab bind to human C5 in a concentration-dependent manner.

In order to determine whether the fusion protein of one embodiment has an anti-VEGF effect, the binding of the fusion protein and the human VEGF 165 protein was determined through an enzyme immunoassay, and the binding affinity of PRO236, PRO237, PRO238, PRO239, PRO241, PRO242, and PRO017 to human VEGF 165 was determined through ELISA.

Specifically, the human VEGF 165 protein was immobilized on a plate, and PRO236, PRO237, PRO238, PRO239, PRO241, PRO242, PRO017, and aflibercept as a control group were allowed to bind. Next, the anti-human immunoglobulin G antibody and the anti-horseradish peroxidase (HRP) antibody were sequentially allowed to bind.

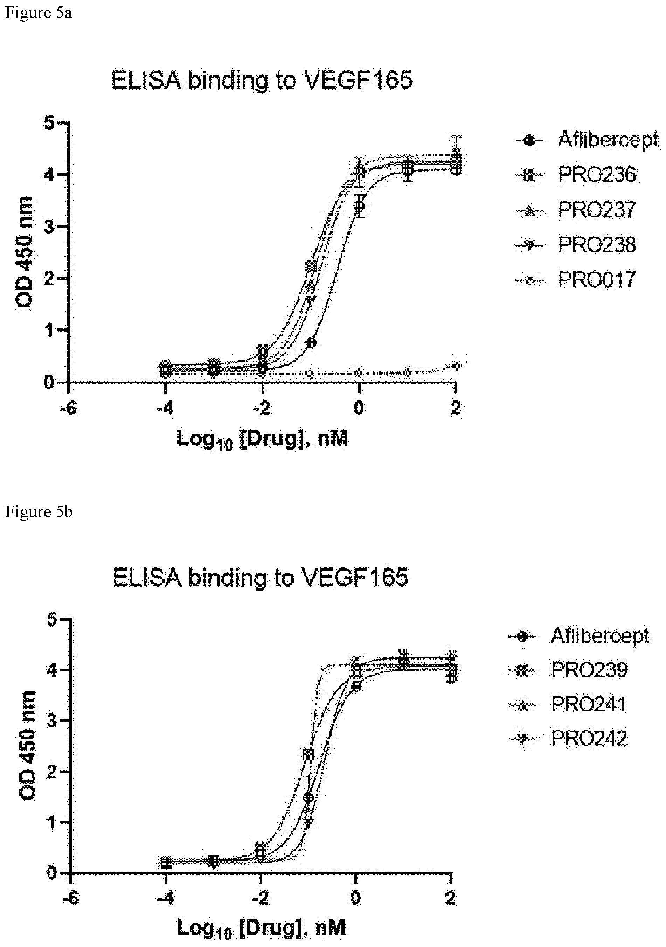

As a result, as shown in Figures 5a and 5b, it was found that through an enzyme immunoassay, aflibercept, PRO236, PRO237, PRO238, PRO239, PRO241, and PRO242, which have anti-VEGF, bind to human VEGF 165 in a concentration-dependent manner.

### Experimental Example 2. Analysis of alternative complement pathway inhibitory effect through hemolysis assay

In order to determine whether the fusion protein dimer of one embodiment inhibits the alternative complement pathway, KNP-301, MOR09611, MOR09675, PRO236, PRO237, PRO239, PRO241, PRO242, S77, and eculizumab were subjected to hemolysis assay (AH50). Specifically, 0.5 mL of rabbit red blood cells was washed with TBS using a centrifuge at 400 xg for 10 minutes. This process was repeated twice, and then the rabbit red blood cells were washed again with GVB EGTA buffer using a centrifuge at 400 xg for 10 minutes. Thereafter, the concentration of rabbit red blood cells was adjusted to 1 × 10⁹ cells/mL using GVB EGTA buffer.

In order to analyze AH50 through the sensitized red blood cells, 12% human C1q-depleted serum was added to a 96-well plate (50 µL/well). In addition, it was treated with KNP-301, MOR09611, MOR09675, PRO236, PRO237, PRO239, PRO241, PRO242, S77, and eculizumab at various concentrations. After incubation at 4 °C for 30 minutes, the rabbit red blood cells were added (2 × 10⁶/well, 50 µL/well). The cells were cultured at 37 °C for 1.5 hours and centrifuged at 600 xg for 10 minutes. 110 µL of supernatant was collected, and then the OD415 value was measured.

The alternative complement pathway inhibitory effects of KNP-301, MOR09611, MOR09675, PRO236, PRO237, PRO239, PRO241, PRO242, S77, and eculizumab was identified through AH50 hemolysis assay.

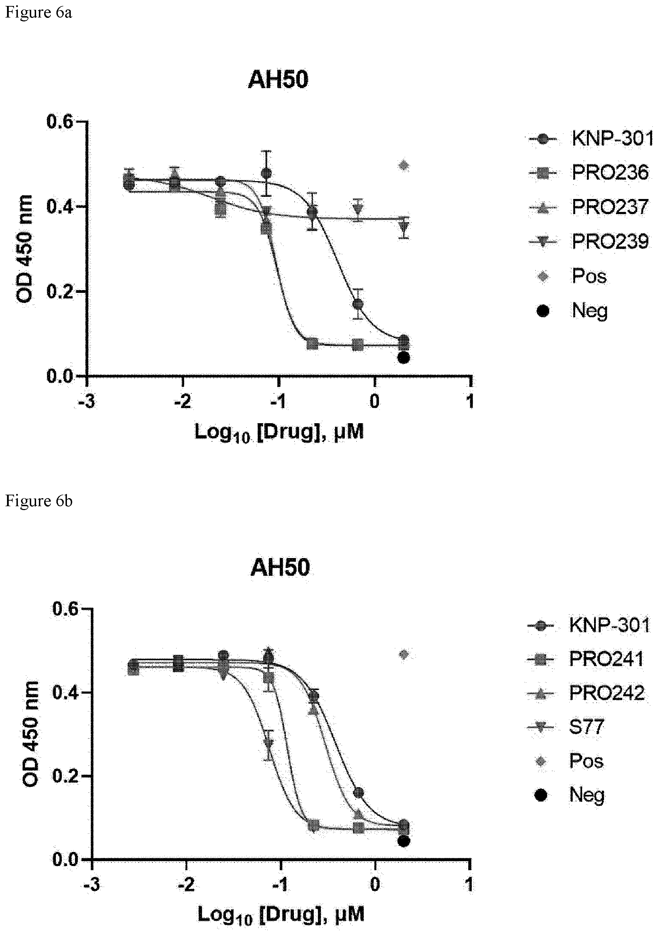

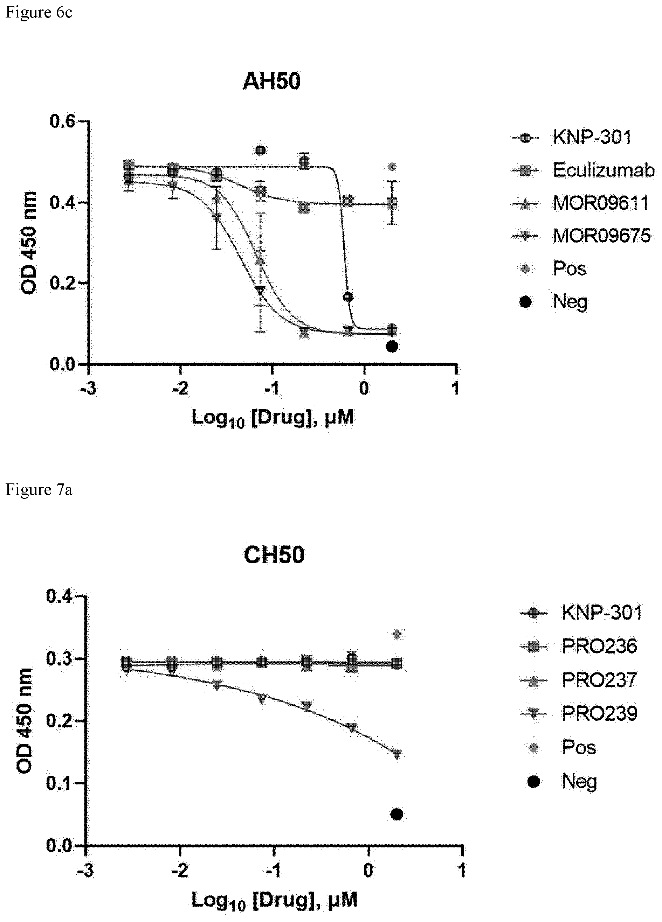

As a result, as shown in Figures 6a to 6c, it was found that KNP-301, MOR09611, MOR09675, PRO236, PRO237, PRO239, PRO241, PRO242, S77, and eculizumab inhibit hemolysis by the alternative complement pathway in a concentration-dependent manner.

### Experimental Example 3. Analysis of classical complement pathway inhibitory effect through hemolysis assay

In order to determine whether the fusion protein dimer of one embodiment does not inhibit the classical complement pathway, KNP-301, MOR09611, MOR09675, PRO236, PRO237, PRO239, PRO241, PRO242, S77, and eculizumab were subjected to hemolysis assay (CH50).

Specifically, the Ab-sensitized red blood cells of sheep were centrifuged at 400 xg for 10 minutes in TBS, and this process was repeated twice, and then the red blood cells of sheep were washed with GVB++ buffer using a centrifuge at 400 xg for 10 minutes. Next, the sensitized red blood cells of sheep were adjusted to a concentration of 1 × 10⁹ cells/mL using GVB++ buffer.

In order to analyze CH50 through the sensitized red blood cells, 4.5% human factor B-depleted serum was added to a 96-well plate (50 µL/well), and then treated with KNP-301, MOR09611, MOR09675, PRO236, PRO237, PRO239, PRO241, PRO242, S77, and eculizumab at various concentrations. After incubation at 4 °C for 30 minutes, the sensitized red blood cells of sheep were added (2.5 × 10⁶/well, 50 µL/well). The cells were cultured at 37 °C for 30 minutes. 110 µL of supernatant was collected by centrifugation at 600 xg for 10 minutes, and then the OD415 value was measured.

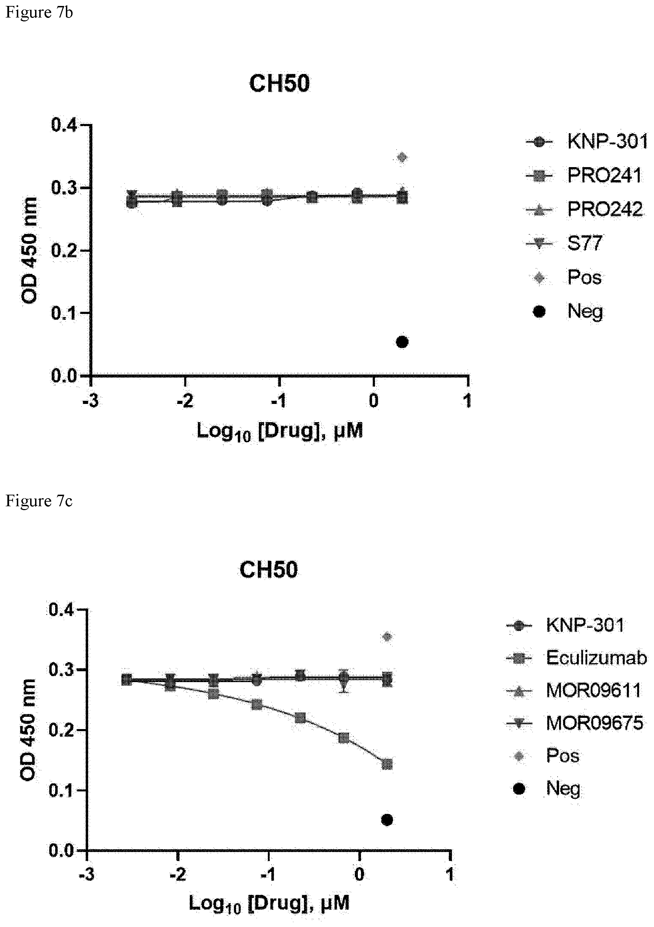

As a result, as shown in Figures 7a to 7c, it was found that KNP-301, MOR09611, MOR09675, PRO236, PRO237, PRO241, PRO242, and S77 do not inhibit hemolysis by the classical complement pathway, but PRO239 and eculizumab inhibit hemolysis.

### Experimental Example 4. Analysis of efficacy of fusion protein dimer using VEGF reporter cells

In order to determine whether the fusion protein dimer of one embodiment effectively inhibits the VEGF protein, it was determined whether it inhibits the binding between VEGF and VEGF receptor.

The VEGF signaling inhibitory effect of aflibercept, PRO236, PRO237, PRO238, PRO239, PRO241, and PRO242 was identified using the reporter cells. Specifically, using VEGF reporter cells (GA3001, Promega, USA), which generate luminescent light by receptor-mediated signaling when VEGF binds, it was confirmed by the degree of luminescence whether aflibercept, PRO236, PRO237, PRO238, PRO239, PRO241, PRO242, and PRO017 inhibit the binding between VEGF and VEGF receptor.

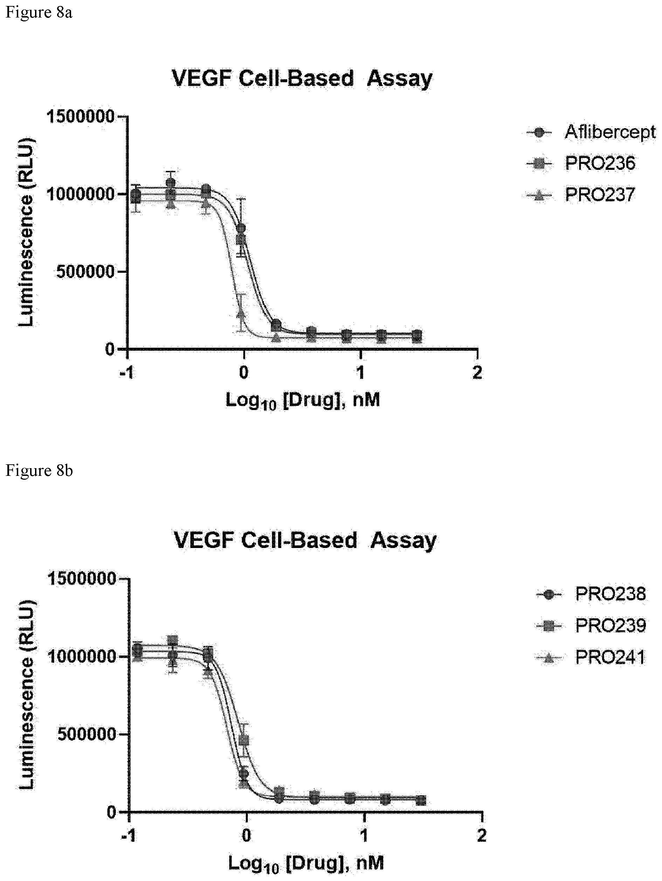

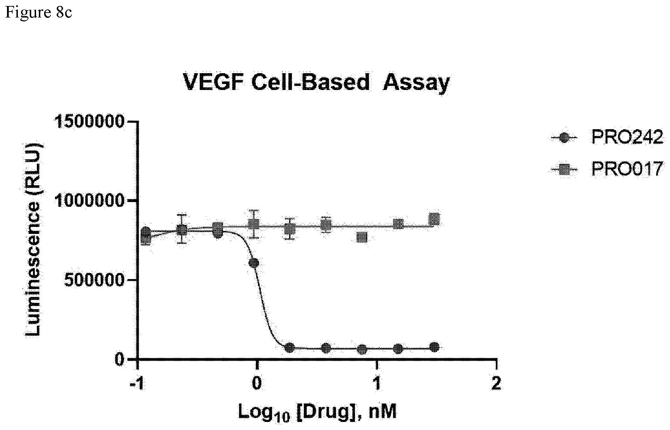

As a result, as shown in Figures 8a to 8c, it was found that aflibercept, PRO236, PRO237, PRO238, PRO239, PRO241, and PRO242 inhibit receptor-mediated signaling by VEGF in a concentration-dependent manner.

## Claims

1. A fusion protein comprising an antibody fragment that specifically binds to C3b (complement component 3b) or C5 (complement component 5); and a protein that specifically binds to VEGF (vascular endothelial growth factor).

2. The fusion protein according to claim 1, wherein the antibody fragment that specifically binds to C3b or C5; and the protein that specifically binds to VEGF are linked via a linker.

3. The fusion protein according to claim 2, wherein the linker is a peptide linker, an immunoglobulin fragment, or a combination thereof.

4. The fusion protein according to claim 3, wherein the immunoglobulin fragment comprises a DANG mutation.

5. The fusion protein according to claim 3, wherein the immunoglobulin fragment comprises any one of the amino acid sequences of SEQ ID NOs: 62 to 68.

6. The fusion protein according to claim 1, wherein the protein that specifically binds to VEGF comprises an antibody that specifically binds to VEGF or a fragment thereof; or the extracellular domain of the VEGF receptor.

7. The fusion protein according to claim 6, wherein the VEGF receptor is VEGF receptor 1 or VEGF receptor 2.

8. The fusion protein according to claim 7, wherein the extracellular domain of the VEGF receptor comprises the amino acid sequence of SEQ ID NO: 52.

9. The fusion protein according to claim 1, wherein the fusion protein consists of the following structural formula (I) or (II):
N'-X-[linker (1)]n-Fc domain or variant thereof-[linker (2)]m-Y-C' (I)
N'-Y-[linker (1)]n-Fc domain or variant thereof-[linker (2)]m-X-C' (II)
in the structural formulas (I) and (II),
N' is the N-terminus of the fusion protein,
C' is the C-terminus of the fusion protein,
X is the antibody fragment that specifically binds to C3b or C5,
Y is the protein that specifically binds to VEGF,
the linkers (1) and (2) are peptide linkers, and
n and m are each independently 0 or 1.

10. The fusion protein according to claim 9, wherein the linker (1) comprises any one of the amino acid sequences of SEQ ID NOs: 53 to 57.

11. The fusion protein according to claim 9, wherein the linker (2) comprises any one of the amino acid sequences of SEQ ID NOs: 58 to 61.

12. The fusion protein according to claim 9, wherein X is Fab or scFv that specifically binds to C3b or C5.

13. The fusion protein according to claim 12, wherein the Fab or scFv that specifically binds to C3b comprises:
i) a heavy chain variable region comprising HCDR1 of SEQ ID NO: 20, HCDR2 of SEQ ID NO: 21, and HCDR3 of SEQ ID NO: 22; and a light chain variable region comprising LCDR1 of SEQ ID NO: 23, LCDR2 of SEQ ID NO: 24, and LCDR3 of SEQ ID NO: 25;
ii) a heavy chain variable region comprising HCDR1 of SEQ ID NO: 28, HCDR2 of SEQ ID NO: 29, and HCDR3 of SEQ ID NO: 30; and a light chain variable region comprising LCDR1 of SEQ ID NO: 31, LCDR2 of SEQ ID NO: 32, and LCDR3 of SEQ ID NO: 33; or
iii) a heavy chain variable region comprising HCDR1 of SEQ ID NO: 36, HCDR2 of SEQ ID NO: 37, and HCDR3 of SEQ ID NO: 38; and a light chain variable region comprising LCDR1 of SEQ ID NO: 39, LCDR2 of SEQ ID NO: 40, and LCDR3 of SEQ ID NO: 41.

14. The fusion protein according to claim 13, wherein the Fab or scFv that specifically binds to C3b comprises:
i) a heavy chain variable region comprising the amino acid sequence of SEQ ID NO: 26 and a light chain variable region comprising the amino acid sequence of SEQ ID NO: 27;
ii) a heavy chain variable region comprising the amino acid sequence of SEQ ID NO: 34 and a light chain variable region comprising the amino acid sequence of SEQ ID NO: 35; or
iii) a heavy chain variable region comprising the amino acid sequence of SEQ ID NO: 42 and a light chain variable region comprising the amino acid sequence of SEQ ID NO: 43.

15. The fusion protein according to claim 12, wherein the Fab or scFv that specifically binds to C5 comprises:
a heavy chain variable region comprising HCDR1 of SEQ ID NO: 44, HCDR2 of SEQ ID NO: 45, and HCDR3 of SEQ ID NO: 46; or
a light chain variable region comprising LCDR1 of SEQ ID NO: 47, LCDR2 of SEQ ID NO: 48, and LCDR3 of SEQ ID NO: 49.

16. The fusion protein according to claim 15, wherein the Fab or scFv that specifically binds to C5 comprises a heavy chain variable region comprising the amino acid sequence of SEQ ID NO: 50 and a light chain variable region comprising the amino acid sequence of SEQ ID NO: 51.

17. The fusion protein according to claim 1, wherein the fusion protein comprises the polypeptide of SEQ ID NO: 2 and the polypeptide of SEQ ID NO: 9; the polypeptide of SEQ ID NO: 4 and the polypeptide of SEQ ID NO: 10; the polypeptide of SEQ ID NO: 6 and the polypeptide of SEQ ID NO: 11; the polypeptide of SEQ ID NO: 8 and the polypeptide of SEQ ID NO: 12; the polypeptide of SEQ ID NO: 13; the polypeptide of SEQ ID NO: 14; the polypeptide of SEQ ID NO: 15; or the polypeptide of SEQ ID NO: 16.

18. A fusion protein dimer in which the two fusion proteins according to any one of claims 1 to 17 are attached to each other.

19. The fusion protein dimer according to claim 18, wherein the fusion protein dimer is a homodimer.

20. A polynucleotide encoding the fusion protein according to any one of claims 1 to 17.

21. A vector comprising the polynucleotide according to claim 20.

22. A host cell transformed with the vector according to claim 21.

23. A pharmaceutical composition for treating or preventing eye diseases, comprising the fusion protein according to claim 1; or the fusion protein dimer according to claim 19 as an active ingredient.

24. The pharmaceutical composition for treating or preventing eye diseases according to claim 23, wherein the eye disease is any one selected from the group consisting of age-related macular degeneration (AMD), geographic atrophy (GA), choroidal neovascularization (CNV), uveitis, diabetic and other ischemia-related retinopathy, diabetic macular edema, pathologic myopia, von Hippel-Lindau disease, ocular histoplasmosis, central retinal vein occlusion (CRVO), corneal neovascularization, and retinal neovascularization.

25. The pharmaceutical composition for treating or preventing eye diseases according to claim 23, wherein the pharmaceutical composition further comprises a pharmaceutically acceptable carrier.
